# EUROPEAN PATENT SPECIFICATION

(11) **EP 0 322 854 B1**
(45) Date of publication and mention of the grant of the patent: **13.07.1994**
(21) Application number: 88121712.9
(22) Date of filing: 27.12.1988
(51) Int. Cl.: C07D 473/00, C07D 473/18, C07D 239/28, C07D 303/12, A61K 31/505, A61K 31/52

(54) **Purinyl cyclobutanes**
Puringruppen enthaltende Cyclobutane
Cyclobutanes contenant un groupe purinyle

(30) Priority: 28.12.1987 US 138737
(43) Date of publication of application: 05.07.1989
(73) Proprietor: E.R. Squibb & Sons, Inc., Princeton, N.J. 08540-4000 (US)
(72) Inventor: Zahler, Robert, Princeton New Jersey (US); Jacobs, Glenn Anthony, Princeton New Jersey (US)
(74) Representative: VOSSIUS & PARTNER

(56) References cited:
- EP-A- 0 160 573
- US-A- 4 605 659
- US-A- 4 617 304
- US-A- 4 636 509
- J. Med. Chem.,1988,vol.31,no.12,pp.2304-2315
- J. Med. Chem.,1988,vol.31,no.9,pp.1798-1804
- JOURNAL OF MEDICINAL CHEMISTRY, vol. 27, no. 10, October 1984 R. VINCE et al. "Carbocyclic Analoges of Xylofuranosylpurine Nucleosides. Synthesis and Antitumor activity." pages 1358-1360

## Description

The present invention relates to purinyl cyclobutanes having antiviral activity.
US-A-4 617 304 is directed to antiviral agents of the formula 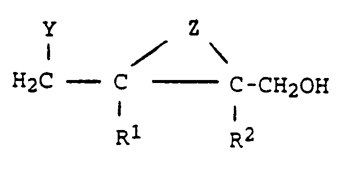 wherein Y is a purin-9-yl or a pyrimidin-1-yl or heterocyclic isostere thereof, R¹ is hydrogen or alkyl of 1 to 4 carbons, R² is hydrogen, alkyl of 1 to 4 carbons, or -CH₂0H,

and Z is 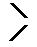 CH₂ or >0.
US-A-4 605 659 disclose cyclopentyl antiviral agents of the formula 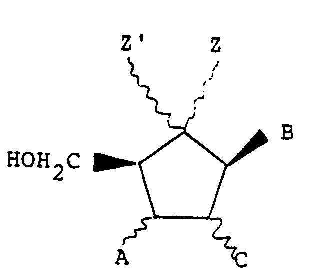 wherein B is a purinyl or pyrimidinyl moiety, A is hydroxy, C is hydrogen or hydroxy, Z is hydrogen and Z' is hydrogen or fluoro or Z and Z' are both fluoro, or Z together with Z' is oxo.

Antiviral activity is exhibited by compounds having the formula 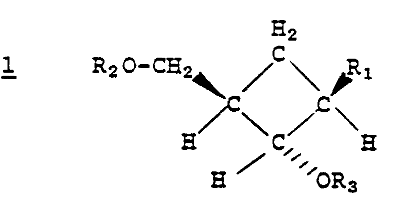 and pharmaceutically acceptable salts thereof. In formula I, and throughout the specification, the symbols are as defined below.
R₁ is 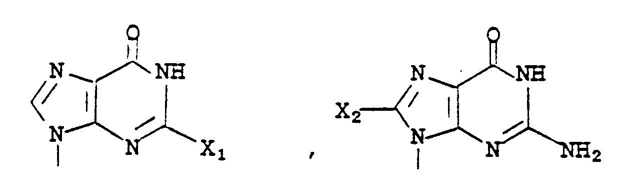 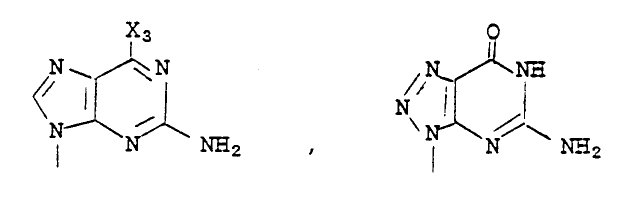 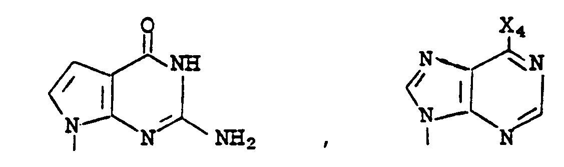 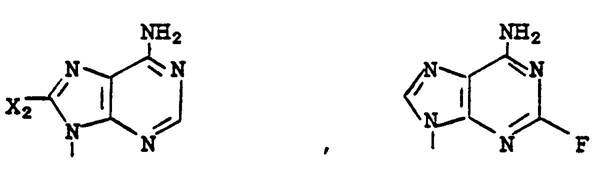 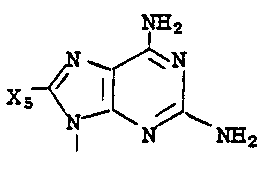 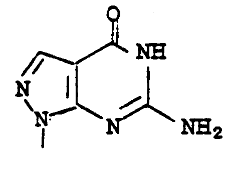 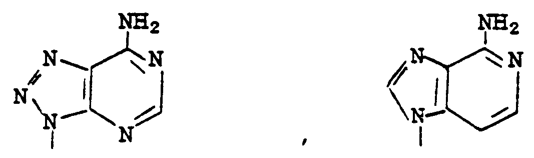 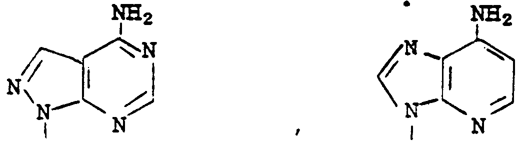 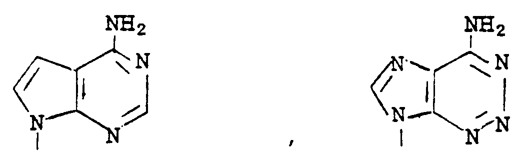 wherein X₁ is hydrogen, amino, 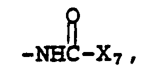 and -N=CHN(X₈)₂
   X₂ is methyl, fluoro, chloro, bromo, iodo, hydroxy, or amino,
   X₃ is hydrogen, chloro, or O-X₈,
   X₄ is amino, 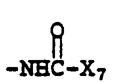 or -N=CHN(X₈)₂,
   X₅ is hydrogen, methyl, fluoro, chloro, bromo, iodo, hydroxy, or amino,
   X₇ is hydrogen, alkyl, substituted alkyl, or aryl,
   X₈ is alkyl,
   R₂ and R₃ are independently hydrogen, -P0₃H₂, or

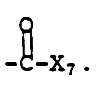

Preferred compounds of formula 1 are wherein R₁ is

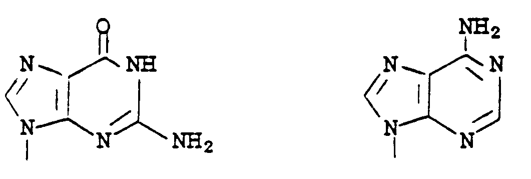

The term "alkyl" refers to both straight and branched chain groups. Those groups having 1 to 10 carbons are preferred. The term "substituted alkyl" refers to alkyl groups having one or more substituents. Preferred substituents are halogen, amino, azido, hydroxy, cyano, trialkylammonium (wherein each alkyl group has 1 to 6 carbons), alkoxy of 1 to 6 carbons, aryl and carboxy. The term "aryl" refers to phenyl and phenyl substituted with one, two or three substituents. Preferred substitutents are alkyl of 1 to 6 carbons, alkoxy of 1 to 6 carbons, halogen, trifluoromethyl, amino, alkylamino, dialkylamino, nitro, cyano, alkanoyloxy of 2 to 11 carbons, carboxy, carbamoyl and hydroxy.

The compounds of formula 1, and the pharmaceutically acceptable salts thereof, are antivital agents that can be used to treat viral infection in mammalian species such as domesticated animals (e.g., dogs, cats, horses and the like) and humans, and avian species (e.g., chickens and turkeys). The compounds of formula 1 wherein R₁ is 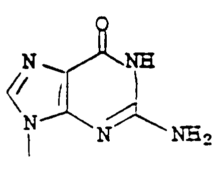 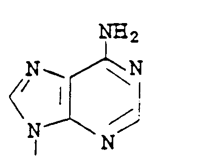 and R₂ and R₃ are independently hydrogen, -P0₃H₂, or 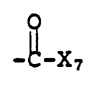 are effective against herpes simplex virus 1 and 2, varicella-zoster virus, cytomegalovirus and vaccinia virus. They may also be effective against a variety of retroviruses and other DNA viruses. Exemplary DNA viruses in addition to those named above include other herpes viruses (e.g., Epstein-Barr virus, pseudorabies virus, and the like), other poxviruses (e.g., monkey pox and myxoma), papovaviruses (e.g., the papilloma viruses), hepatitis B virus, and adenoviruses. Exemplary retroviruses are those effecting man, such as human immunodeficiency viruses (HIV) and human T-cell lymphotropic viruses I and II (HTLV-I and II), and those affecting other animals, such as feline leukemia virus, murine leukemia virus, and equine infectious anemia virus.

For internal infections, the compounds may be administered orally or parenterally in an amount effective to treat the infection. The dosage will, of course, depend on the severity of the infection, but will likely be in the range of about 1.0 to 30 mg/kg of body weight.

For infections of the eye, or other external tissues, (e.g., mouth and skin) the compositions may be applied to the infected part of the body of the patient topically as an ointment, cream, aerosol, gel, powder, lotion, suspension or solution (e.g., as eye drops). The concentration of the compound in the vehicle will, or course, depend on the severity of the infection, but will likely be in the range of about 0.1 to 7% by weight.

The compounds of this invention can be prepared from the known chemical compound 1-chloro-3-(hydroxymethyl)cyclobutane, which is a racemic mixture of cis and trans diasteriomers. Its hydroxymethyl group is first protected using, for example, a silyl containing group (e.g., a hindered trisubstituted silyl such as t-butyldiphenylsilyl, di-t-butylmethylsilyl, or triisopropylsilyl), trityl, substituted trityl (e.g., 4-monomethoxytrityl or 4,4'-dimethoxytrityl), or benzyl protecting group. The protection reaction yields a compound of the formula 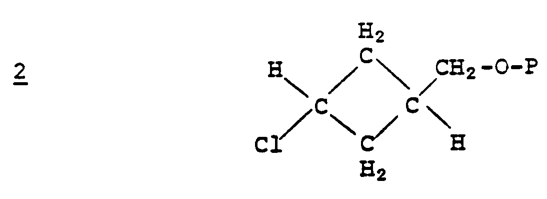 wherein the protecting group "P" serves to protect the hydroxyl group from involvement in subsequent reactions. This protected cyclobutane is a mixture of cis and trans isomers.

Protection with a benzyl group can be accomplished by treating 1-chloro-3-(hydroxymethyl) cyclobutane with sodium hydride in the presence of benzyl bromide in a polar aprotic solvent such as dimethylformamide, dimethylsulfoxide or tetrahydrofuran. Protection with a t-butyldiphenylsilyl group can be accomplished by treating a dimethylformamide solution of 1-chloro-3-(hydroxymethyl)cyclobutane with t-butyldiphenylsilyl chloride in the presence of imidazole. Protection with a trityl or substituted trityl group can be accomplished by (i) treating a pyridine solution of 1-chloro-3-(hydroxymethyl)cyclobutane with trityl chloride or substituted trityl chloride, (ii) treating a dimethylformamide solution of 1-chloro-3-(hydroxymethyl)cyclobutane with trityl chloride or substituted trityl chloride in the presence 4-N,N-dimethylaminopyridine or (iii) treating a dichloromethane solution of 1-chloro-3-(hydroxymethyl)cyclobutane with trityl chloride or substituted trityl chloride in the presence of triethylamine.

Basic elimination of hydrogen chloride from a compound of formula using a base such as potassium t-butoxide in a polar aprotic solvent, such as dimethylsulfoxide or tetrahydrofuran yields the corresponding compound having the formula 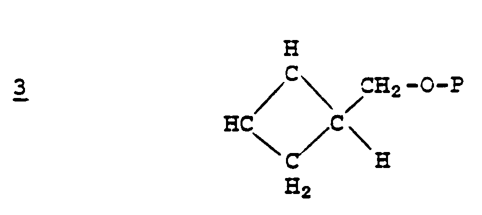 as a racemic mixture. Alternatively, a base such as lithium diisopropylamide in a solvent such as tetrahydrofuran can be used to effect the elimination.

Epoxidation of a compound of formula using a peracid, such as m-chloroperoxybenzoic acid yields the corresponding compound having the formula 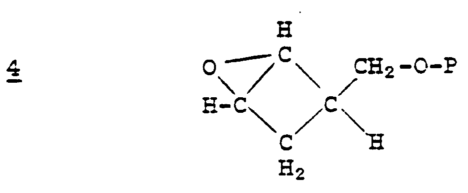 as a racemic mixture of cis and trans diastereomers. Separation of the diastereomers using conventional methodology provides the desired trans stereoisomer having the formula 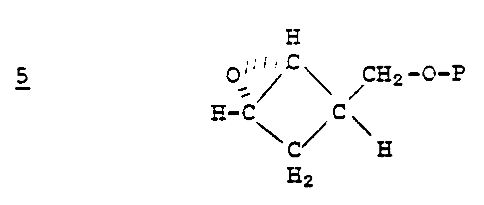 as a racemic mixture. Alternatively, preferential formation of the trans epoxide can be achieved by treating a methanol solution of a compound of formula with benzonitrile/30% hydrogen peroxide in the presence of a buffer (e.g., potassium bicarbonate or monobasic potassium phosphate/sodium hydroxide).

Nucleophilic substitution on the epoxide of a compound of formula 5 using a compound of formula 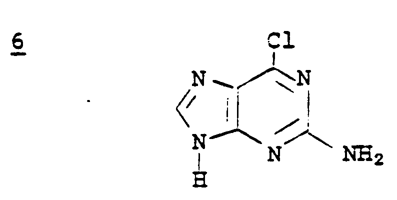 in the presence of a base such as potassium carbonate, sodium hydride, or potassium hydride in an aprotic polar solvent such as dimethylformamide, dimethyl sulfoxide, or sulfolane (tetramethylene sulfone) yields the corresponding compound of formula

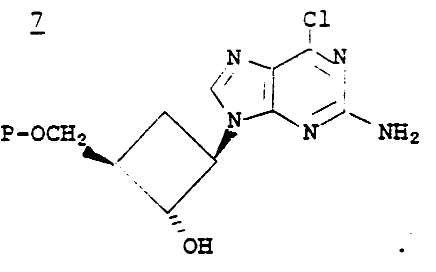

Optionally, the reaction can be run in the presence of a metal chelating catalyst such as 18-crown-6 (1,4,7,10,13,16-hexaoxacyclooctadecane) or 15-crown-5 (1,4,7,10,13-pentaoxacyclopentadecane).

Removal of the protecting group P from a compound of formula 7 yields a compound of formula wherein R₁ is 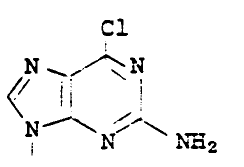 and R₂ and R₃ are hydrogen. When the protecting group P is benzyl, the group can be removed by treatment with boron trichloride in dichloromethane. When the protecting group P is a silyl protecting group, removal of the group can be accomplished using fluoride ion (e.g., tetrabutylammonium fluoride in tetrahydrofuran). When the protecting group P is a trityl or substituted trityl group, removal of the group can be accomplished using aqueous acid (e.g., aqueous acetic acid) according to methods known in the art.

Reaction of a compound of formula 5 with a protected form of guanine such as a compound of formula 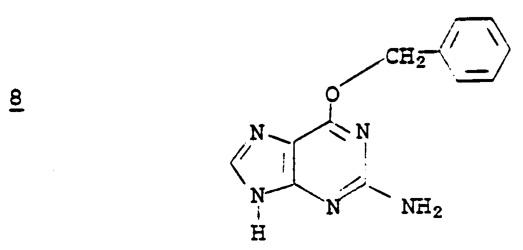 under conditions analogous to those used in the preparation of compound 7 provides a compound of formula

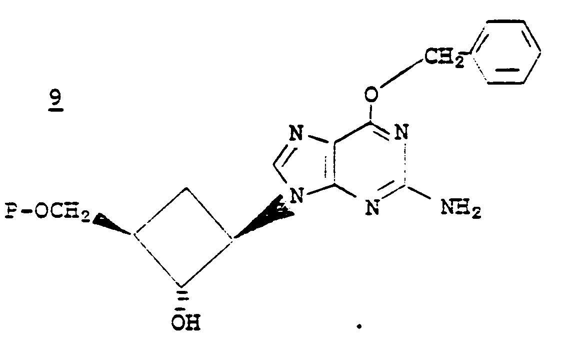

Removal of the protecting groups from a compound of formula yields a compound of formula wherein R₁ is 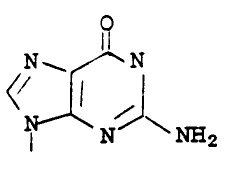 and R₂ and R₃ are hydrogen.

When the protecting group P in is benzyl, simultaneous removal of the P group and the purine O-benzyl group can be effected by using sodium in liquid ammonia, by hydrogenolysis (e.g., palladium hydroxide on carbon, cyclohexene, and ethanol), or by using boron trichloride in dichloromethane. Alternatively, the purine O-benzyl group can be removed first using aqueous alcoholic mineral acid followed by removal of the P group using, for example, sodium in liquid ammonia or hydrogenolysis. When the protecting group P is a silyl protecting group, removal of the P group can be accomplished using fluoride ion (e.g., tetrabutylammonium fluoride in tetrahydrofuran). The purine O-benzyl group can then be removed with aqueous alcoholic mineral acid, by hydrogeneolysis, or with sodium in liquid ammonia. Alternatively, the purine O-benzyl group can be deprotected first by hydrogenolysis followed by removal of the silyl P group using fluoride ion. When the protecting group P is a trityl or substituted trityl group, removal of the P group and the purine O-benzyl group can be accomplished simultaneously using aqueous/alcoholic mineral acid.

Alternatively this compound of formula can be prepared from a compound of formula 7. For example, when the protecting group P in 7 is benzyl, removal of the P group can be effected first by treatment with boron trichloride, and then the chloro group can be hydrolized using aqueous acid (e.g., aqueous hydrochloric acid). Alternatively, the chloro group can be hydrolized first followed by removal of the benzyl group. When the protecting group P in 7 is silyl, the protecting group can be removed by treatment with fluoride ion, and then the chloro group can be hydrolized. When the protecting group P in 7 is a trityl or substituted trityl group, the protecting group can be removed and the chloro group can be simultaneously hydrolized using aqueous acid.

Alternatively this compound of formula can be prepared by removal of the protecting group P from a compound of formula 7 followed by treatment with adenosine deaminase by methods known in the art (e.g., M. J. Robins and P. W. Hatfield, Can. J. Chem., 60, 547 (1982)).

A compound of formula wherein R₁ is 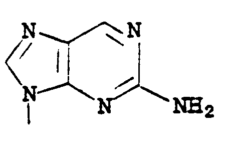 and R₂ and R₃ are hydrogen can be prepared from a compound of formula 7. For example, when the P group in 7 is benzyl, deprotection and reduction of the chloro group can be accomplished simultaneously by hydrogenation (e.g., ammonium formate and palladium on carbon in methanol; palladium hdyroxide on carbon and cyclohexene in ethanol; or palladium on carbon, hydrogen and ethanol). When the P group is silyl the chloro group can first be reduced by hydrogenation and then the protecting group can be removed using fluoride ion. Alternatively, the silyl protecting group can be removed first and then the chloro group can be reduced. When the P group is trityl or substituted trityl, deprotection of the P group can be effected using aqueous acid (e.g., aqueous acetic acid) and the chloro group can then be reduced.

Alternatively, this compound of formula can be prepared by reacting an optionally protected compound of formula 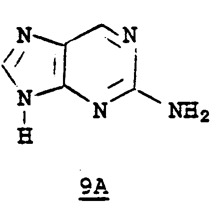 with a compound of formula according to the procedures analogous to those used in the preparation of a compound of formula 7, followed by removal of the protecting group(s) by methods known in the art. The optionally protected forms of compound 9A can be protected at the amino (-NH₂) group by such exemplary groups as acyl (e.g., acetyl or benzoyl), trityl, or substituted trityl.

A compound of formula wherein R₁ is 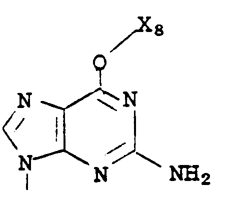 and R₂ and R₃ are hydrogen can be prepared from a compound of formula 7 or from a compound of formula 1 wherein R₁ is 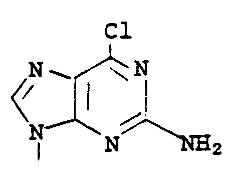 and R₂ and R₃ are hydrogen by methods known in the art. See, for example, J.F. Gerster, et al., J. Amer. Chem. Soc., 87, 3752 (1965); K.K. Ogilvie, et al., Can. J. Chem., 62, 2702 (1984); M. R. Harnden, et al., J. Med. Chem., 30, 1636 (1987).

Alternatively, this compound of formula can be prepared by reacting a compound of formula 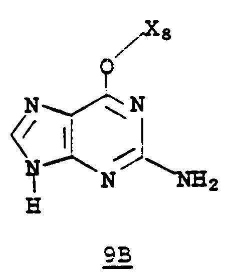 with a compound of formula according to the procedures analogous to those used in the preparation of a compound of formula 7, followed by removal of the protecting group P by methods known in the art. Compounds of formula 9B can be prepared from the compound of formula by methods known in the art (see, e.g., W. A. Bowles et.al., J. Med. Chem.,6, 471 (1963)).

A compound of formula wherein R₁ is 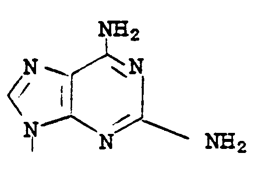 and R₂ and R₃ are hydrogen can be prepared from a compound of formula 7 by methods known in the art. (See e.g., J. C. Martin , et. al., J. Med. Chem., 28, 358 (1985)). Thus for example, when a compound of formula 7 (wherein P is a protecting group such as benzyl, silyl, trityl or substituted trityl) is treated with hot methanolic ammonia, displacement of the chloro group with an amino group will result. When the protecting group P is a benzyl group, subsequent deprotection can be accomplished by hydrogeneolysis, by sodium in liquid ammonia, or by using boron trichloride. When the protecting group P is a silyl group, subsequent deprotection can be accomplished using fluoride ion. When the protecting group is a trityl or substituted trityl group, subsequent deprotection can be accomplished using an aqueous acid.

Alternatively, this compound of formula can be prepared by reacting an optionally protected compound of formula 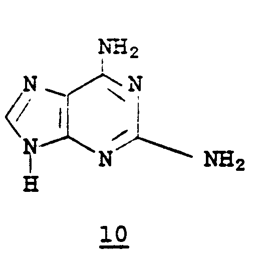 with a compound of formula according to the procedures analogous to those used in the preparation of a compound of formula 7, followed by removal of the protecting group(s) by methods known in the art. The optionally protected forms of compound 10 can be protected at the amino (-NH₂) groups by such exemplary groups as acyl (e.g. acetyl or benzoyl), trityl, or substituted trityl. When the amino protecting groups are acyl, removal of the acyl groups can be accomplished first using catalytic sodium methoxide in methanol or methanolic ammonia, and then the P protecting group can be removed, for example, by hydrogenolysis when P is benzyl, by treatment with fluoride ion when P is silyl, or by aqueous acid when P is trityl or substituted trityl. Alternatively, the silyl, benzyl or trityl protecting group P could be removed first followed by removal of the acyl protecting groups. When all of the protecting groups are trityl or substituted trityl, simultaneous deprotection of all of the trityl groups can be accomplished using aqueous acid. When the amino protecting groups are trityl and P is benzyl, the trityl groups can be removed first with aqueous acid and the benzyl group can then be removed by hydrogenolysis, sodium in liquid ammonia, or boron trichloride.

Reaction of a compound of formula 5 with an optionally protected compound of formula 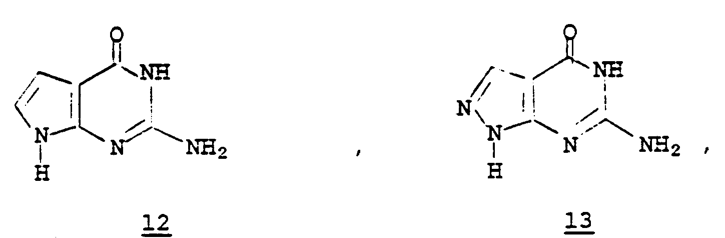 or 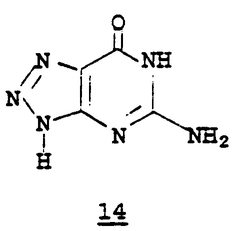 in the presence of a base such as potassium carbonate, sodium hydride, or potassium hydride in a polar aprotic solvent (e.g., dimethylformamide, dimethyl sulfoxide or sulfolane), in the optional presence of 18- crown-6 or 15-crown-5, gives after subsequent removal of the protecting group(s), the corresponding compound of formula wherein R₁ is 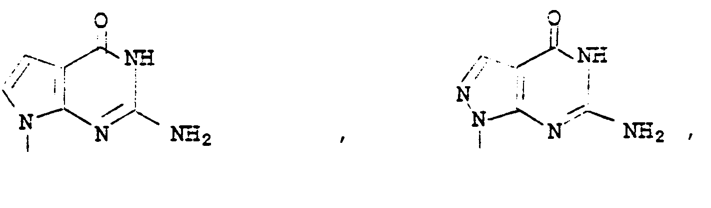 or 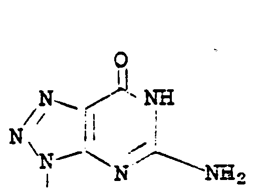 and R₂ and R₃ are hydrogen.

The optionally protected forms of compounds 12, 13 and 14 can be protected at the amino (-NH₂) group by such exemplary groups as acyl (e.g. acetyl or benzoyl), trityl, or substituted trityl. These protecting groups can be removed by methods known in the art.

Alternatively, these compounds of formula 1 can be prepared by reaction of 5 with a compound of the formula 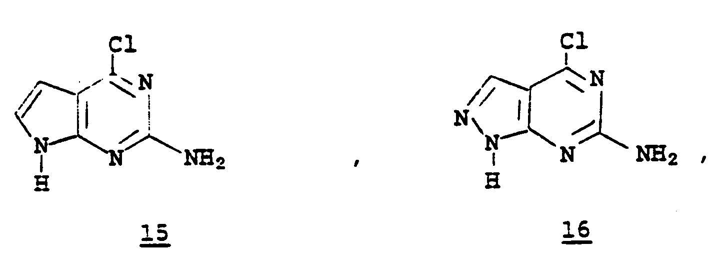 or 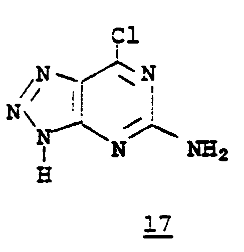 respectively, by procedures analogous to those used in the preparation of 7 , followed by acid hydrolysis of the chloro group and simultaneous or subsequent removal of the protecting group P.

Reaction of a compound of formula 5 with a compound of formula 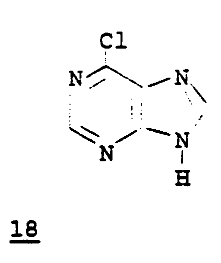 by methodology analogous to that used to prepare a compound of formula 7 yields a compound of formula 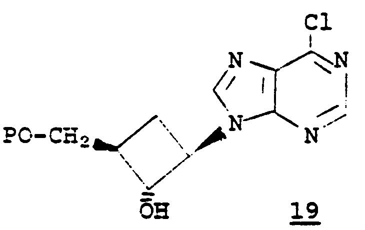 Treatment of a compound of formula 19 with methanolic ammonia and subsequent removal of the protecting group P, yields the compound of formula wherein R₁ is 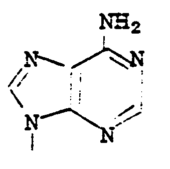 and R₂ and R₃ are hydrogen.

Alternatively, this compound of formula 1 can be prepared by reaction of a compound of formula 5 with a compound of formula 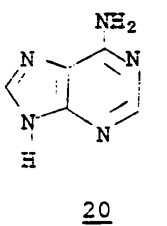 by methodology analogous to that used to prepare a compound of formula 7 and subsequent removal of the protecting group P.
If the protecting group P is a benzyl group, this group can be removed by hydrogenolysis (e.g., palladium hydroxide on carbon, cyclohexene, ethanol) or by using sodium in liquid ammonia.

Reaction of the compound of formula 5 with a compound of formula 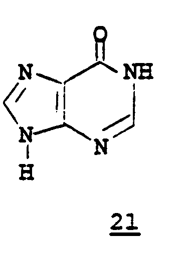 by methodology analogous to that used to prepare a compound of formula 7 and subsequent removal of the protecting group P yields the corresponding compound of formula wherein R₁ is 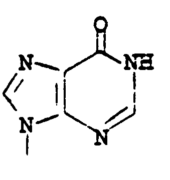 and R₂ and R₃ are hydrogen.

Alternatively, this compound of formula 1 can be prepared by treatment of the compound of formula 1 wherein R₁ is 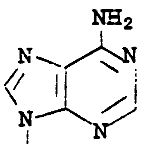 and R₂ and R₃ are hydrogen with adenosine deaminase or nitrous acid.

Reaction of the compound of formula 5 with a compound of formula 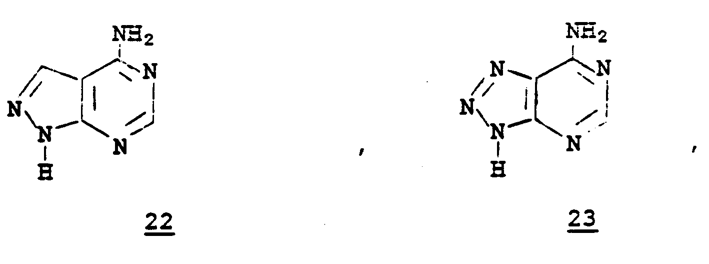 or 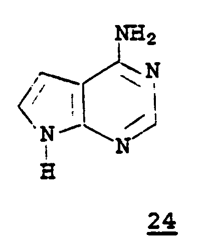 or a protected form thereof, by methodology analogous to that used to prepare a compound of formula 7 and subsequent removal of the protecting groups, yields the corresponding compound of formula 1 wherein R₁ is 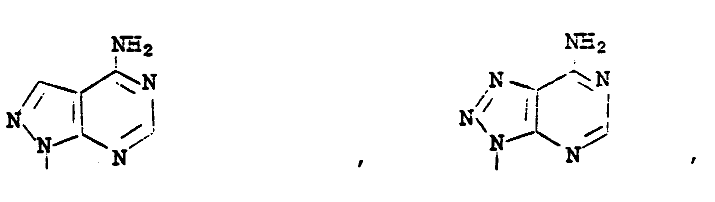 or 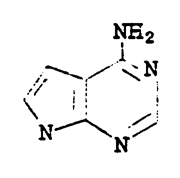 and R₂ and R₃ are hydrogen.

The protected forms of compounds 22, 23 and 24 can be protected at the amino (-NH₂) group by such exemplary groups as acyl (e.g. acetyl or benzoyl), trityl, or substituted trityl. The protecting groups can then be removed by methods known in the art.

Alternatively, these compounds of formula 1 can be prepared by reaction of a compound of formula 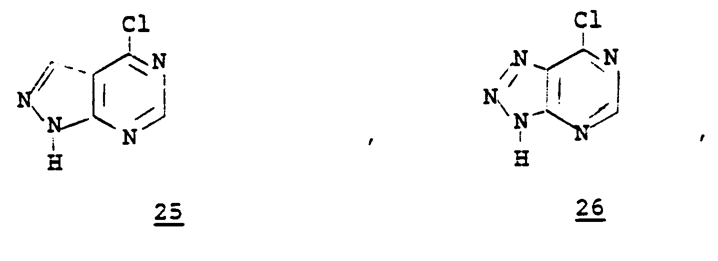 or 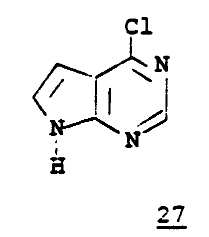 with a compound of formula 5 by methods analogous to those used in the preparation of a compound of formula 7. This affords the corresponding compounds of formula 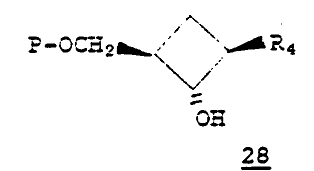 wherein R₄ is 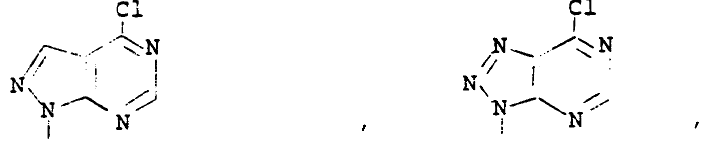 or 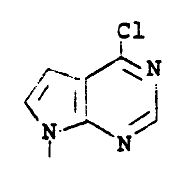 Treatment of a compound of formula 28 with hot ammonia in an alcohol and subsequent deprotection of the P protecting group yields the compound of formula wherein R₁ is 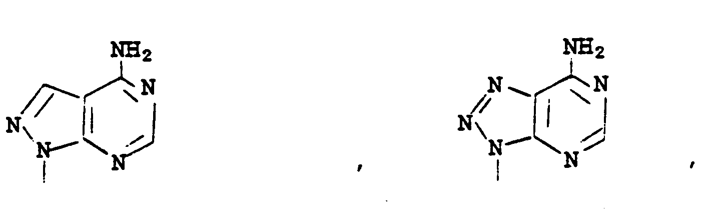 or 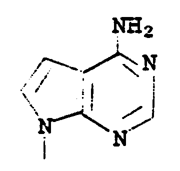 and R₂ and R₃ are hydrogen.

Reaction of a compound having the formula with an optionally protected compound having the formula 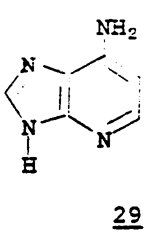 following methodology analogous to that used to prepare a compound of formula 7, and subsequent removal of the protecting group(s), yields the corresponding compound of formula wherein R₁ is 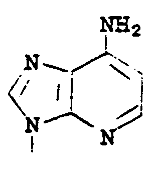 and R₂ and R₃ are hydrogen.

The optionally protected forms of compound 29 are compounds wherein the amino (-NH₂) group is protected by such exemplary groups as acyl (e.g. acetyl or benzoyl), trityl or substituted trityl. These groups can then be removed by methods known in the art.

The compound of formula wherein R₁ is 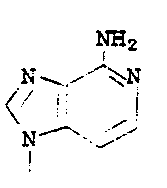 and R₂ and R₃ are hydrogen can be prepared by reaction of a compound of formula 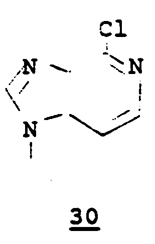 with a compound of formula 5, according to the procedures analogous to those used in the preparation of compound 7, which affords an intermediate compound of formula 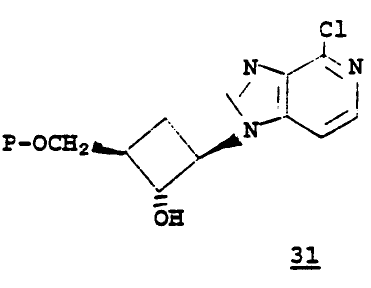 Subsequent treatment of the compound of formula 31 with hydrazine, followed by Raney nickel reduction, using methods known in the art (e.g., R. I. Glazer, et al., Biochem. Pharmacol., 35, 4523 (1986); R. J. Rousseau, et al., Biochemistry, 5, 756 (1966)) yields a compound of formula 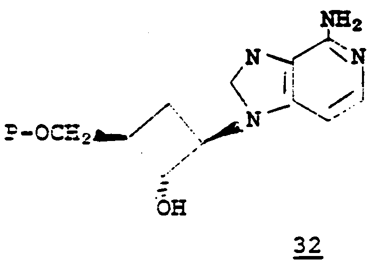 The protecting group P in 32 can then be removed by methods known in the art. Alternatively, the protecting group P in 31 can be removed first and the corresponding deprotected compound can then be treated with hydrazine followed by Ranay nickel reduction. For example, when P is benzyl, deprotection of 31 can be achieved with boron trichloride. When P is silyl, trityl, or substituted trityl deprotection can be achieved by methods known in the art.

The compound of formula wherein R₁ is 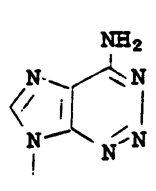 and R₂ and R₃ are hydrogen can be prepared from a compound of formula wherein R₁ is 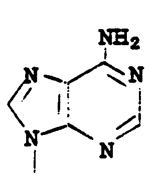 and R₂ and R₃ are hydrogen by methodology known in the art (e.g, the conversion of adenosine to 2- azaadenosine; J. A. Montgomery et al. in "Nucleic Acid Chemistry" Part 2, L.B. Townsend and R. S. Tipson, Eds., John Wiley and Sons, p. 681, 1978).

Compounds of formula 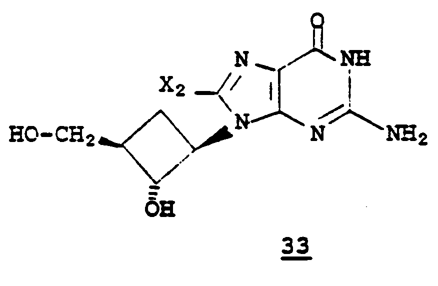 wherein X₂ is methyl, chloro, bromo, iodo, hydroxy, or amino can be prepared starting from the compound of formula 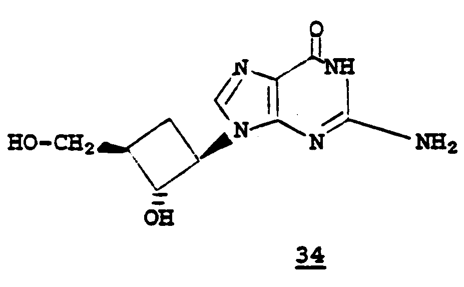 by methods known in the art (e.g. M. J. Robins, et al., J. Med. Chem.; 27, 1486 (1984)).

For example, treatment of the compound of formula 34 with t-butylhydroperoxide in aqueous sulfuric acid in the presence of ferrous sulfate provides the compound of formula 33 wherein X₂ is methyl. Treatment of the compound of formula 34 with dry hydrogen chloride and m-chloroperoxybenzoic acid in dimethylformamide provides the compound of formula 33 wherein X₂ is chloro. Treatment of the compound of formula 34 with iodine monochloride in aqueous methanol provides a compound of formula 33 wherein X₂ is iodo. Treatment of the compound of formula 34 with bromine water provides the compound of formula 33 wherein X₂ is bromo. Treatment of the compound of formula 33 wherein X₂ is bromo with refluxing aqueous hydrazine provides the compound of formula 33 wherein X₂ is amino. Treatment of the compound of formula 33 wherein X₂ is bromo with sodium acetate/acetic acid provides the compound of formula 33 wherein X₂ is hydroxyl.

A compound of formula 33 wherein X₂ is fluoro can be prepared from the compound of formula 33 wherein X₂ is bromo or iodo and where the amino (-NH₂) and/or hydroxyl groups are optionally protected with acyl (e.g. acetyl or benzoyl) groups. Treatment with fluoride ion (e.g., sodium or potassium fluoride in a solvent such as dimethylformamide or tetrabutylammonium fluoride in tetrahydrofuran) followed by removal of the optional acyl protecting groups using, for example, catalytic sodium methoxide in methanol or methanolic ammonia provides the compound of formula 33 wherein X₂ is fluoro.

Compounds of formula 1 wherein R₁ is 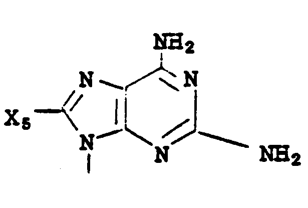 and X₅ is methyl, chloro, bromo, iodo, hydroxy, or amino and R₂ and R₃ are hydrogen can be prepared from the corresponding compound of formula 1 wherein X₅, R₂ and R₃ are hydrogen using procedures known in the art. See, for example, R. E. Holmes, et al., J. Amer. Chem. Soc., 86, 1242 (1964); R. E. Holmes, et al., J. Amer. Chem. Soc., 87, 1772 (1965); R. A. Long, et al., J. Med. Chem., 27, 1486 (1984). The amino (-NH₂) and/or hydroxyl groups in the compound of formula 1, wherein X₅, R₂ and R₃ are hydrogen, can be optionally protected by acyl (e.g. acetyl or benzoyl) groups prior to replacement of the X₅ hydrogen by a methyl group. Subsequent deprotection can be accomplished by treatment with sodium methoxide in methanol or methanolic ammonia.

The compound of formula 1 wherein R₁ is 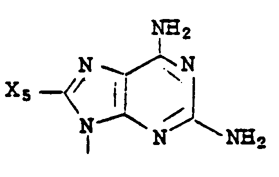 and X₅ is fluoro and R₂ and R₃ are hydrogen can be prepared from the corresponding compound of formula 1 wherein X₅ is bromo or iodo and R₂ and R₃ are hydrogen. The amino (-NH₂) and/or hydroxyl groups can be optionally protected with acyl (e.g. acetyl or benzoyl) groups. Treatment with fluoride ion (e.g., sodium or potassium fluoride in a solvent such as dimethylformamide or tetrabutylammonium fluoride in tetrahydrofuran) followed by removal of the optional acyl protecting groups (using, for example, catalytic sodium methoxide in methanol or methanolic ammonia) provides the compound of formula wherein R₁ is 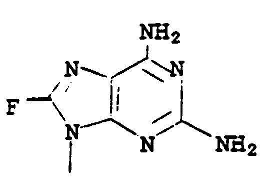 and R₂ and R₃ are hydrogen.

Compounds of formula wherein R₁ is 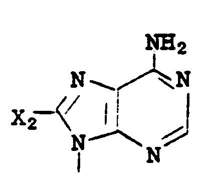 and X₂ is methyl, chloro, bromo, iodo, hydroxy, or amino and R₂ and R₃ are hydrogen can be prepared from the corresponding compound of formula 1 wherein R₁ is 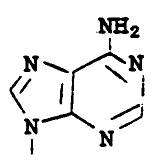 and R₂ and R₃ are hydrogen following procedures known in the art. See, for example, R. E. Holmes, et. al., J. Amer. Chem. Soc., 86, 1242 (1964); R. E. Holmes, et. al., J. Amer. Chem. Soc., 87, 1772 (1965); R. A. Long et. al., J. Org. Chem., 32, 2751 (1967).

A compound of formula 1 wherein R₁ is 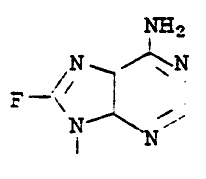 and R₂ and R₃ are hydrogen can be prepared from a compound of formula 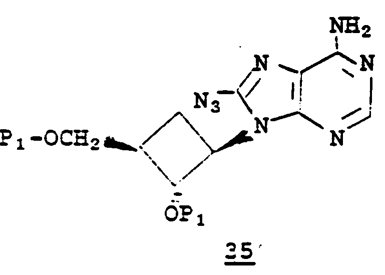 wherein P₁ is an acyl protecting group, (for example, acetyl or benzoyl) by methodology known in the art (e.g., M. Ikehara, et al., Chem. Commun., 1509 (1968)). The compound of formula 35 can be prepared by known methods from the compound of formula 1 wherein R₁ is 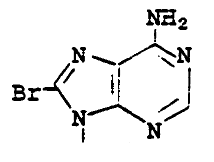 and R₂ and R₃ are hydrogen by treatment with sodium azide followed by acylation of the hydroxyl groups. See, for example, R. A. Long, et al., J. Org. Chem., 32, 2751 (1967).

The compound of formula 1 wherein R₁ is 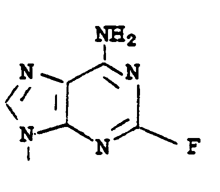 and R₂ and R₃ are hydrogen can be prepared from the corresponding compound of formula 1 wherein R₁ is 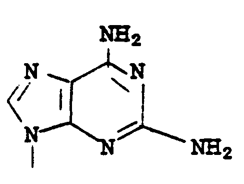 and R₂ and R₃ are hydrogen by following known procedures. See, for example J.A. Montgomery, et al. in "Synthetic Procedures in Nucleic Acid Chemistry", Vol. 1, W. W. Zorbach and R.S. Tipson, Eds., Interscience Publishers (John Wiley and Sons), N.Y., p. 205, 1968.

Compounds of the formula wherein R₁ is 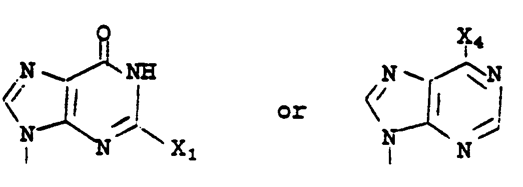 X₁ and X₄ are 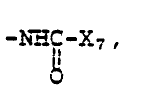 and R₂ and R₃ are hydrogen or 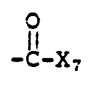 (or wherein X₁ and X₄ are amino (-NH₂), and one or both of R₂ and R₃ are 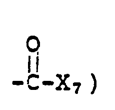 can be prepared from the corresponding compounds of formula 1 wherein X₁ and X₄ are amino and R₂ and R₃ are hydrogen by methods known in the art.

All of the other compounds of formula 1 wherein one or both of R₂ and R₃ are 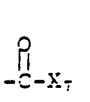 can be prepared by methods known in the art from the corresponding compounds of formula 1 wherein R₂ and R₃ are hydrogen.

For examples of acylation procedures, see "Synthetic Procedures in Nucleic Acid Chemistry", Vol. 1, W. W. Zorbach and R. S. Tipson, Eds., John Wiley and Sons, 1968; "Nucleic Acid Chemistry," Part 1, L. B. Townsend and R. S. Tipson, Eds., John Wiley and Sons, 1978; Y. Ishido, et al., Nucleosides and Nucleotides, 5, 159 (1986); J. C. Martin, et al., J. Pharm. Sci., 76, 180 (1987); A. Matsuda, et al., Synthesis, 385 (1986).

Compounds of the formula 1 wherein R₁ is 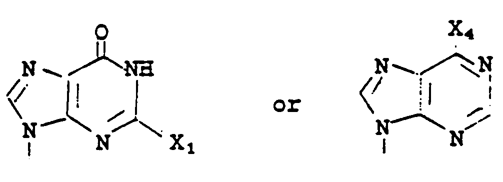 and X₁ and X₄ are -N = CHN(X₈)₂ can be prepared from the corresponding compounds of formula wherein X₁ and X₄ are amino by procedures known in the art. See, for example, A. Holy and J. Zemlicka, Collect. Czech. Chem. Commun., 32, 3159 (1967); K. K. Ogilvie, et al., Nucleosides and Nucleotides, 4, 507 (1985); M. H. Caruthers et al., J. Amer. Chem. Soc., 108, 2040 (1986).

The compounds of formula 1 wherein R₂ and/or R₃ are -P0₃H₂ can be prepared from the corresponding compounds of formula 1 wherein R₂ and R₃ are hydrogen by procedures known in the art. See, for example, H. Schaller, et al., J. Amer. Chem. Soc., 85, 3821 (1963); J. Beres, et al., J. Med. Chem., 29, 494 (1986); R. Noyori, et al., Tet. Lett., 28, 2259 (1987); W. Pfleiderer, et al., Helv. Chim. Acta., 70, 1286 (1987); "Nucleic Acid Chemistry", Part 2, L. B. Townsend and R. S. Tipson, Eds., John Wiley and Sons, 1978.

The compounds of formula 1 wherein R₁ is 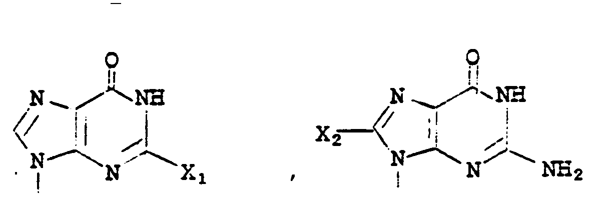 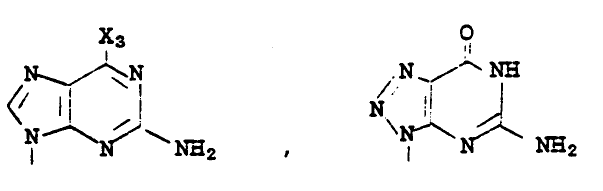 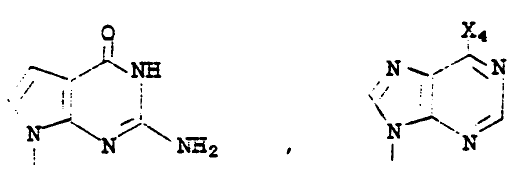 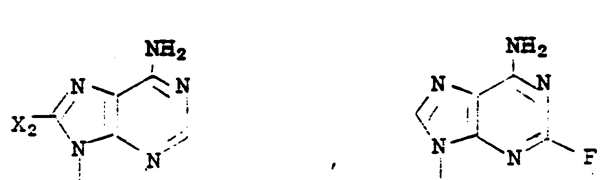 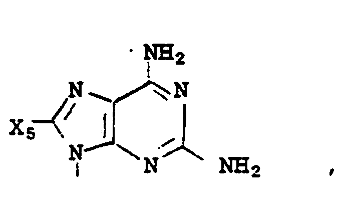 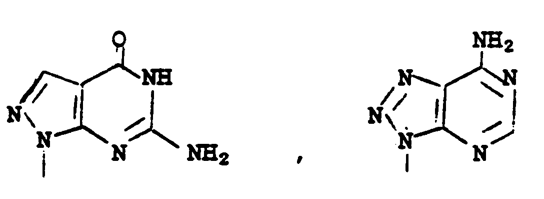 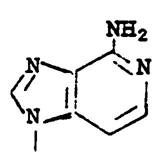 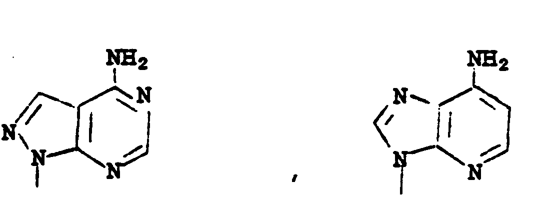 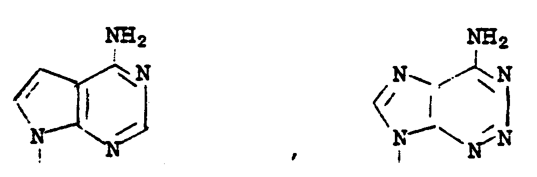 can form acid addition salts with inorganic or organic acids. Illustrative are the hydrohalide (e.g. hydrochloride and hydrobromide), alkylsulfonate, sulfate, phosphate and carboxylate salts.

The compounds of formula I wherein R₁ is 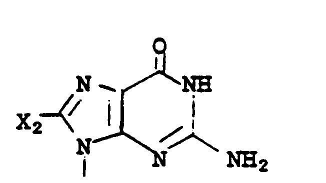 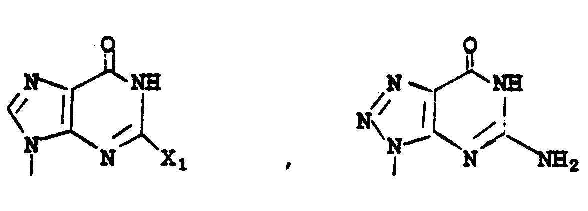 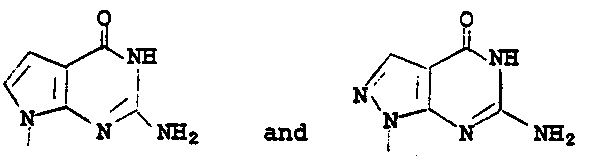 can form basic salts with inorganic and organic. bases. Illustrative are alkali metal salts (e.g., sodium and potassium), alkaline earth metal salts (e.g. calcium and magnesium), ammonium and substituted ammonium salts.

The compounds of formula I wherein R₂ and/or R₃ are -P0₃H₂ can form basic salts with inorganic and organic bases. Illustrative are alkali metal salts (e.g., sodium and potassium), alkaline earth metal salts (e.g., calcium and magnesium), ammonium and substituted ammonium salts.

The stereochemistry shown for the compounds of this invention is relative, not absolute. It is drawn to show that in the compounds of this invention, the base (Ri) is cis with respect to the -CH₂-OR₂ substituent and trans with respect to the OR₃ substituent.

The following examples are specific embodiments of this invention.

### Example 1

### (1α,2β,3α)-9-[2-Hydroxy-3-(hydroxymethyl)cyclobutyl]guanine

### A) [[(3-Chlorocyclobutyl)methoxy]methyl]benzene

A mixture of 3-chlorocyclobutanemethanol (17.3 g, 0.143 mole) and benzylbromide (26.96 g, 0.158 mole) in dry dimethylformamide (123 ml) was stirred at room temperature under an argon atmosphere and a 60% suspension of sodium hydride (6.31 g, 0.158 mole) was added. The reaction was stirred at ambient temperature for 22.5 hours. The reaction mixture was poured into 600 ml of water and the aqueous mixture extracted with ethyl acetate (4 x 500 ml). The ethyl acetate extracts were combined and dried over anhydrous sodium sulfate and the ethyl acetate evaporated in vacuo yielding the crude product as a yellow oil. The material was purified on a 2-liter Merck silica gel column eluting with 3 liters of hexane, followed by 5% ethyl acetate/hexane. The fractions containing the desired product were combined and the volatiles evaporated in vacuo yielding 28.6 g of the title compound as a pale yellow oil.

### B) [(2-Cyclobuten-1-ylmethoxy)methyl]benzene

[[(3-Chlorocyclobutyl)methoxy]methyl]benzene 82 g, 0.39 mole) in 390 ml of dry dimethylsulfoxide was slowly added to a solution of potassium t-butoxide (132 g, 1.17 mole) in 390 ml of dry dimethylsulfoxide in a water-bath at 18 _{°} C under a dry argon atmosphere. After stirring for 1 hour at room temperature, the reaction mixture was poured into 1600 ml of water and extracted with ether (3 x 1000 ml). The combined ether extracts were back-extracted with water (4 x 2000 ml), the extracts were then dried over sodium sulfate, and the volatiles were removed in vacuo. The crude product was divided into two equal portions and each portion was purified on separate 3.5 liter Merck silica columns, eluting with 3% ethyl acetatehexane. Appropriate fractions were combined and the solvents removed in vacuo yielding 60.0 g of the title compound as a colorless liquid.

### C) (la,2a,4a)-2-[(Phenylmethoxy)methyl]-5-oxabicyclo[2.1.0]pentane

A solution of 80% m-chloroperoxybenzoic acid (19.0 g, 0.088 mol) in 600 ml of dichloromethane was cooled to O _{°} C and a solution of [(2-cyclobuten-1-ylmethoxy)methyl]benzene (14.0 g, 0.080 mol) in 50 ml of dichloromethane was added and the resulting mixture was stirred overnight at 5°C under an argon atomosphere. The precipitated m-chlorobenzoic acid was removed by filtration, and the dichloromethane solution was washed with 5% sodium thiosulfate (I x 500 ml), saturated sodium bicarbonate (3 x 500 ml), and water (2 x 500 ml). The dichloromethane solution was then dried over anhydrous sodium sulfate. The solution was filtered and the dichloromethane was evaporated in vacuo yielding 11.6 g of 1:1 mixture of cis and trans product.

A quantity of the cis an trans isomers (1:1) were separated by preparative HPLC using a "Water's Prep 500" with a 500 ml silica gel column eluting with 2.5% ethyl acetate/hexane loading 2 g of mixture at 100 ml/minute and then eluting the column at a flow rate of 200 ml/minute (total 10 g of mixture used). Peak shaving technique was used to enrich one isomer over the other, with the mixture being recycled through the column 3 times. A total of 2.1 g of trans epoxide and 2.48 of cis epoxide were separated.

### Alternative Separation of Cis and Trans Isomers

A crude mixture of cis and trans epoxide (1:1, 58 g) was isolated from two separate m-chloroperoxybenzoic acid oxidations of two 27.05 g batches of [(2-cyclobuten-1-ylmethoxy)methyl] benzene following the general procedure described above. Two equal 29 g portions were purified on two separate 3.5 liter silica gel columns eluting with 5% ethyl acetate/pentane. The fractions containing essentially pure (1a,2a,4a)-2-[-(phenylmethoxy)methyl]-5-oxabicyclo[2.1.0]pentane were combined and the solvents removed in vacuo yielding 4.02 g of desired compound. Those fractions containing a greater than 1:1 ratio of trans epoxide were combined and the solvents removed in vacuo yielding 20.5 g of a mixture enriched in trans epoxide.

The trans-enriched mixture was further purified by preparative HPLC using a "Waters Prep 500" equipped with two tandem 500 ml silica gel column eluting with 5% ethyl acetate/pentane loading 4 g of the mixture at a time (at a flow rate of 250 ml/minute). A total of 20.5 g of material was loaded in this fashion. Peak shaving technique was used to enrich one isomer over the other, with the mixture being recycled back through the column once. Eventually, 6.91 g of essentially pure (1a,2a,4a)-2-[(phenylmethoxy)methyl]-5- oxabicyclo[2.1.0]pentane was isolated in this fashion. Total recovery was 10.93 g.

### Alternative Epoxidation Reaction

To a mixture of benzonitrile (0.80 ml, 7.8 mmol) and potassium bicarbonate (170 mg, 1.7 mmol) in 12 ml of methanol was added [(2-cyclobuten-1-ylmethoxy)methyl]benzene (523 mg, 3.0 mmol) in 12 ml of chloroform followed by the addition of 1 ml of 30% hydrogen peroxide. The mixture was rapidly stirred at room temperature under an argon atmosphere for 92 hours. The reaction was poured into 75 ml of 5% sodium thiosulfate and was extracted with 200 ml of ether. The ether extract was washed with 200 ml of water, 200 ml of saturated sodium bicarbonate and 200 ml of saturated sodium chloride solution. The ether extract was dried over anhydrous sodium sulfate, filtered and the ether removed in vacuo yielding 1.1 grams of crude mixture. The crude material was purified on a 100 ml Merck silica column eluting with 500 ml of hexanes followed by eluting with 1000 ml of 22% ethyl acetate/hexanes. All fractions containing cis and trans-epoxide were combined. The volatiles were removed in vacuo yielding 478 mg of a 1:2.5 mixture of cis and trans isomers, respectively.

### D) (1α,2β,4β)-2[2-Amino-6-(phenylmethoxy)-9H-purine-9-yl]-4-[(phenylmethoxy)methyl]cyclobutanol

Freshly dried (65 °C @ 0.1 mm Hg overnight) 2-amino-6-benzyloxypurine (1.21 g, 5.0 mmol) and (1α,2α,4α)-2-[(phenylmethoxy)methyl]-5-oxabicyclo[2.1.0]-pentane (571 mg, 3.0 mmol) were dissolved in 13 ml of dry dimethylformamide under an argon atmosphere. 60% Sodium hydride (60 mg, 1.5 mmol) was added to the reaction mixture at room temperature and the reaction was then heated at 110_{°}C for 3 days. The reaction was cooled to room temperature and the dimethylformamide was evaporated under vacuum at 40 °C yielding the crude product as a brown solid. The residue was partially dissolved in 8 ml of dichloromethane and purified on a 500 ml Whatman LPS1 silica column eluting with 1500 ml of dichloromethane followed by 300 ml of 2% methanol/dichloromethane collecting 20 ml fractions. The fractions containing pure product were combined and the volatiles removed in vacuo yielding the title compound as a colorless solid, 336 mg.

### Alternative Reaction

To a stirring suspension of (la,2a,4a)-2-[(phenyl-methoxy)methyl]-5-oxabicyclo[2.1.0]pentane (57.1 mg, 0.30 mmol), 2-amino-6-benzyloxypurine (121.0 mg, 0.50 mmol, dried for 24 hours at 80 °C, 1 mm Hg, over P₂O₅), and 18-crown-6 ether (61.0 mg, 0.23 mmol) in sulfolane (1.3 ml, dried over 3° A molecular sieves) at room temperature under argon was added sodium hydride (7.0 mg, 0.175 mmol, 60% oil dispension). After the mixture was heated to 110_{°}C, the solution became homogeneous. After 21 hours at 110_{°}C, the reaction was cooled to room temperature and was quenched with acetic acid (0.025 ml).

Most of the solvent was removed by distillation (0.3 mm Hg) leaving an orange oily residue. This residue was purified by silica gel chromatography (Merck; 230-400 mesh (0.037-0.063 mm)),eluting with CH₂CI₂, 1%, 2%, and then 3% MeOH:CH₂CI₂ to give the pure coupled product (54.8 mg).

### E) (1α,2β,3α)-9-[2-Hydroxy-3-(hydroxymethyl)cyclobutyl]guanine

(1α,2β,4β)-2-[2-Amino-6-(phenylmethoxy)-9H-purin-9-yl]-4-[(phenylmethoxy)methyl]cyclobutanol (336 mg, 0.78 mmol) in 3 ml of dry, distilled tetrahydrofuran was added to 30 ml of liquid ammonia at -78 °C under an argon atmosphere. With stirring, finely cut sodium (165 mg, 7.2 mmol) was added and when the mixture became dark blue in color the cooling bath was removed and the mixture was allowed to stir for 10 minutes. The reaction was quenched by adding small portions of ammonium chloride until the reaction became colorless. The volatiles were then removed by allowing a slow stream of nitrogen to pass through the reaction mixture yielding the crude product as a colorless solid. The crude solid was dissolved in 20 ml of water and the pH was adjusted from pH 12.6 to pH 7.0 by adding 1 N hydrochloric acid solution. When the pH reached 10 the product began to precipitate from solution. The precipitated product was collected by centrifugation and was washed twice with cold water (2 x 4 ml). The resulting colorless solid was dried in vacuo overnight at room temperature yielding 134 mg of the title product, melting point 246 °C (dec.)

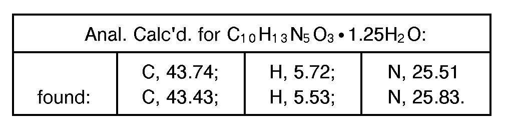

### Example 2

### (1α,2β,3α)-3-(6-Amino-9H-purin-9-yl)-2-hydroxycyclobutanemethanol

### A) (1α,2β,4β)-2-(6-Amino-9H-purin-9-yl)-4-[(phenylmethoxy)methyl]cyclobutanol

A mixture of dried adenine (557 mg, 4.125 mmol) and (la,2a,4a)-2-[(phenylmethoxy)methyl]-5- oxabicyclo[2.1.0]pentane (523 mg, 2.75 mmol; see example 1C) was partially dissolved in 5.5 ml of dry dimethylformamide under an argon atmosphere. To this mixture was added potassium carbonate (95 mg, 0.69 mmol) followed by 18-crown-6 ether (330 mg, 1.25 mmol) and then the mixture was heated at 110_{°}C for 50 hours. The reaction was cooled to room temperature, and the volatiles were removed under vacuum at 40 °C yielding the crude product as a brown solid. The residue was partially dissolved in 10 ml of dichloromethane and purified on a 250 ml Whatman LPS1 silical gel column, eluting with 750 ml of dichloromethane followed by 2000 ml of 22% methanol/dichloromethane. The fractions containing the pure desired product were combined and the volatiles removed in vacuo yielding the title compound as a colorless solid, 212 mg.

### B) (1α,2β,3α)-3-(6-Amino-9H-purin-9-yl)-2-hydroxycyclobutanemethanol

(1α,2β,4β)-2-(6-Amino-9H-purin-9-yl)-4-[(phenylmethoxy)methyl]cyclobutanol (200 mg, 0.615 mmol) was dissolved in 40 ml of absolute ethanol and 20 ml of cyclohexene. 20% Palladium hydroxide (140 mg) was added and the mixture was heated at reflux for 24 hours. At this point, an additional 70 mg of 20% palladium hydroxide catalyst was added, and after another 8 and 10 hours two 70 mg portions of catalyst were again added. After refluxing for a total of 66 hours, the reaction was filtered through a "Millipore" filter to remove the catalyst and the catalyst was washed with approximately 10 ml of ethanol. The volatiles were removed in vacuo yielding the crude product as a colorless solid. The material was dissolved in 5 ml of water and purified on a 50 ml HP-20 column eluting with 600 ml of a 50% acetonitrile-water/water gradient. The fractions containing pure product were combined, the acetonitrile removed in vacuo, and the water lyophilized to yield 59 mg of product as a colorless solid, melting point 240 (dec.).

### Example 3

### (1α,2β,3α)-2-Amino-1,9-dihydro-9-[2-hydroxy-3-(hydroxymethyl)cyclobutyl]-8-methyl-6H-purin-6-one

Nitrogen was bubbled through a solution of (1α,2β,3α)-9-[2-hydroxy-3-(hydroxymethyl)cyclobutyl]-guanine (74 mg; 0.3 mmol) and FeS0₄·7H₂0 (278 mg; 1 mmol) in 16 ml of 1M H₂SO₄ to remove traces of oxygen. After 30 minutes the reaction was blanketed with argon and tertiary butyl hydroperoxide (500 mg, 70% solution in water; 4 mmol) in 3 ml of water was added dropwise over 30 minutes. After stirring for 2 hours, the reaction mixture was neutralized with 1 N NaOH and the resulting dark brown suspension was centrifuged to remove the viscous brown sludge. The clear colorless supernate was loaded onto an HP-20 column (2.5 x 15 cm) which was eluted with 1 liter of water followed by a linear gradient from water to 25% acetonitrile-water. The pure desired fractions were concentrated and lyophilized to afford 37 mg of the title product as a white solid having m.p. 228 - 232 °C (dec.).

### Example 4

### (1α,2β,3α)-2-Amino-8-bromo-1,9-dihydro-9-[2-hydroxy-3-(hydroxymethyl)cyclobutyl]-6H-purin-6-one

(1α,2β,3α)-9-[2-Hydroxy-3-(hydroxymethyl)cyclobutyl]guanine (225 mg; 0.9 mmol) was suspended in water (35 ml) at room temperature. Bromine water (which was prepared by stirring 2 ml of bromine in 75 ml of water and decanting off the supernatant after 10 minutes) (7 ml) was added dropwise over 5 minutes. After this addition, the suspension became a solution for five minutes, and then a precipitate reappeared. TLC (Silica gel, 6:3:1, chloroform: methanol: conc. ammonia) of an aliquot of the suspension dissolved in dimethylformamide showed only partial reaction. only partial reaction. An additional 2 ml of bromine water was added and the TLC showed that a trace of starting material remained. Then 0.5 ml of bromine water was added. After a total reaction time of approximately 1 hour, the reaction mixture was cooled to 0 _{°} C. The solid was filtered, washed with cold water, and dried to afford 292 mg, of crude title product. A 120 mg portion was recrystallized from hot water to afford 112 mg, of the title product as an off white solid having m.p. >240 _{°} C.

### Example 5

### (1α,2β,3α)-2,8-Diamino-1,9-dihydro-9-[2-hydroxy-3-(hydroxymethyl)cyclobutyl]-6H-purine-6-one.

(1 α,2β,3α)-2-Amino-8-bromo-1,9-dihydro-9-[2-hydroxy-3-(hydroxymethyl)cyclobutyl]-6H-purine-6-one (155 mg; 0.47 mmol) was refluxed in 7 ml of water and 0.36 ml of hydrazine-hydrate for 168 hours (7 days). After each 24 hour period, 0.2 ml of hydrazine-hydrate and 0.5 ml of water were added to the refluxing suspension. The solvents were removed in vacuo and the white solid residue was triturated with water to remove inorganics. The residue was loaded onto an HP-20 column (2.5 x 45 cm) which was eluted with a continuous gradient from water to water-dimethylformamide, 1:1. The pure product containing fractions were concentrated to a white powder. This powder was suspended in water and filtered to remove residual dimethylformamide. Drying in vacuo for 18 hours over CaS0₄ afforded 50 mg of the title product as a white powder having m.p. 225 °C (dec.).

## Claims (Claims for the following Contracting State(s) : AT, BE, CH, DE, FR, GB, IT, Ll, LU, NL, SE)

1. A compound having the formula 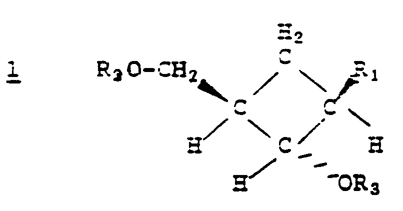 or a pharmaceutically acceptable salt thereof, wherein R₁ is 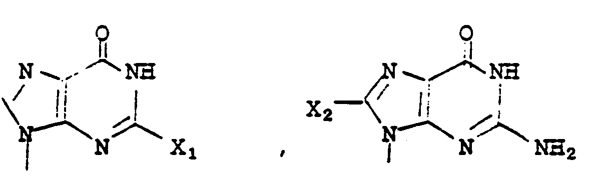 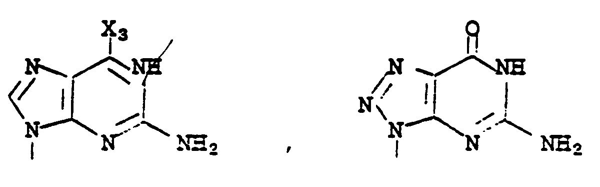 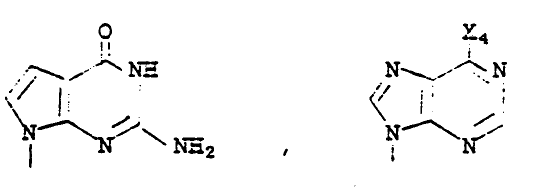 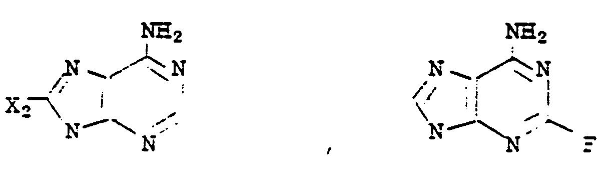 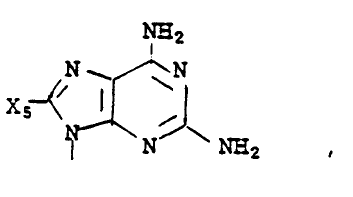 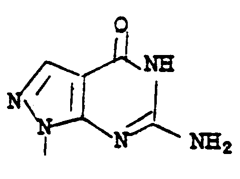 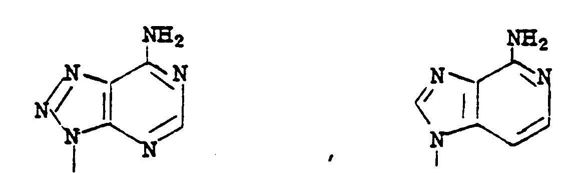 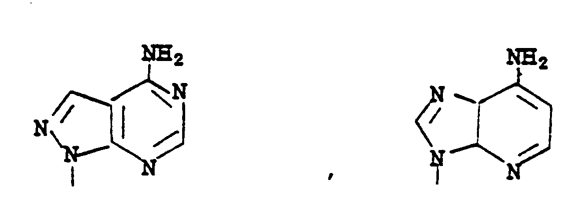 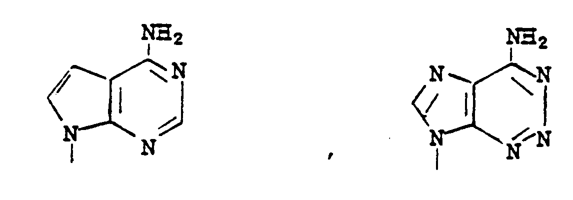 wherein X₁ is hydrogen, amino, 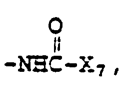 and -N=CHN(X₈)₂
X₂ is methyl, fluoro, chloro, bromo, iodo, hydroxy, or amino,
X₃ is hydrogen, chloro, or O-X₈,
X₄ is amino, 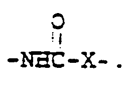 or -N=CHN(X₈)₂,
X₅ is hydrogen, methyl, fluoro, chloro, bromo, iodo, hydroxy, or amino,
X₇ is hydrogen, alkyl, substituted alkyl, or aryl,
X₈ is alkyl,
R₂ and R₃ are independently hydrogen, -P0₃H₂, or 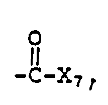 wherein
the term "alkyl" refers to both straight and branched chain groups having 1 to 10 carbons, the term "substituted alkyl" refers to alkyl groups having one or more halogen, amino, azido, hydroxy, cyano, trialkylammonium (wherein each alkyl group has 1 to 6 carbons), alkoxy of 1 to 6 carbons, aryl and carboxy substituents; the term "aryl" refers to phenyl and phenyl substituted with one, two or three alkyl of 1 to 6 carbons, alkoxy of 1 to 6 carbons, halogen, trifluoromethyl, amino, alkylamino, dialkylamino, nitro, cyano, alkanoyloxy of 2 to 11 carbons, carboxy, carbamoyl and hydroxy substituents.

2. A compound in accordance with claim 1 wherein R₁ is 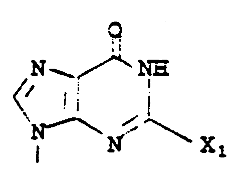 or

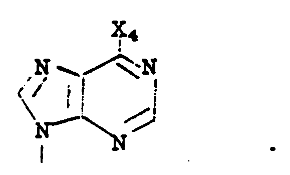

3. A compound in accordance with claim 2 wherein R₁ is

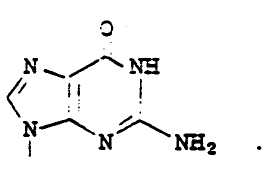

4. A compound in accordance with claim 2 wherein R₁ is

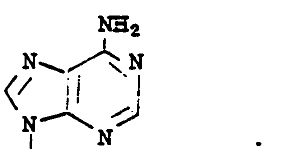

5. A compound in accordance with any one of claims 1, 3 or 4 wherein R₂ and R₃ are independently hydrogen or

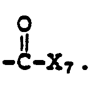

6. A compound in accordance with any one of claims 1, 3 or 4 wherein R₂ and R₃ are independently hydrogen or -P0₃H₂.

7. A compound in accordance with claim 6 wherein R₂ and R₃ are hydrogen.

8. A compound in accordance with claim 1 wherein R₁ is

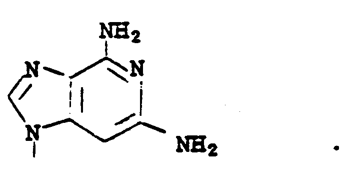

9. A compound in accordance with claim 1 wherein R₁ is

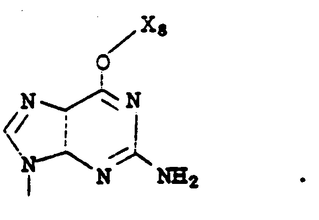

10. A compound in accordance with claim 1 wherein R₁ is

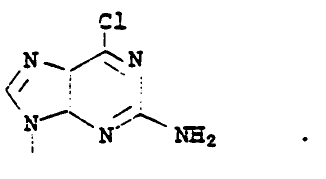

11. A compound in accordance with claim 1 wherein R₁ is

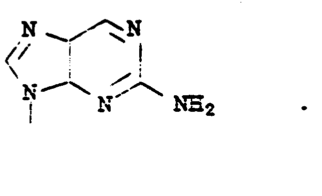

12. A compound in accordance with claim 1 wherein R₁ is

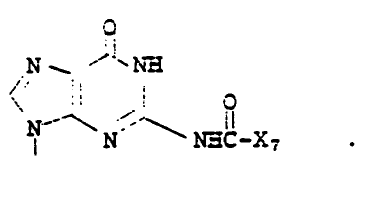

13. A compound in accordance with claim 1 wherein R₁ is

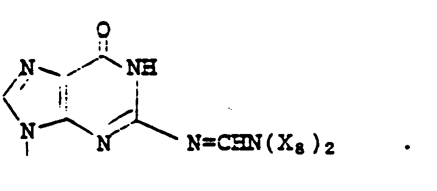

14. A compound in accordance with claim 1 wherein R₁ is

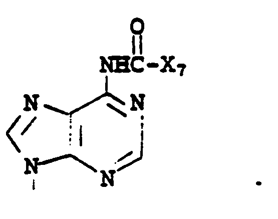

15. A compound in accordance with claim 1 wherein R₁ is

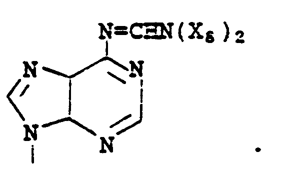

16. The compound in accordance with claim 1, (1α,2β,3α)-9-[2-hydroxy-3-(hydroxymethyl)cyclobutyl]-guanine.

17. The compound in accordance with claim 1, (1α,2β,3α)-3-(6-amino-9H-purin-9-yl)-2-hydrox- ycyclobutanemethanol.

18. The compound in accordance with claim 1, (1α,2β,3α)-2-amino-1,9-dihydro-9-[2-hydroxy-3-(hydroxymethyl)cyclobutyl]-8-methyl-6H-purin-6-one.

19. The compound in accordance with claim 1, (1α,2β,3α)-2-amino-8-bromo-1,9-dihydro-9-[2-hydroxy-3-(hydroxymethyl)cyclobutyl]-6H-purin-6-one.

20. The compound in accordance with claim 1, (1α,2β,3α)-2,8-diamino-1,9-dihydro-9-[2-hydroxy-3-(hydroxymethyl)cyclobutyl]-6H-purine-6-one.

21. A compound as defined in any one of claims 1 to 20 for use as a pharmaceutically active agent.

22. A compound as defined in any one of claims 1 to 20 for use in a method of treating a viral infection in a mammalian or avian host.

23. A pharmaceutical composition comprising an effective amount of a compound as defined in any one of claims 1 to 20 and a physiologically acceptable carrier.

24. An antiviral composition comprising an effective amount of a compound as defined in any one of claims 1 to 20 and a physiologically acceptable carrier.

25. The use of a compound as defined in any one of claims 1 to 20 for preparing an antiviral composition.

26. A compound having the formula 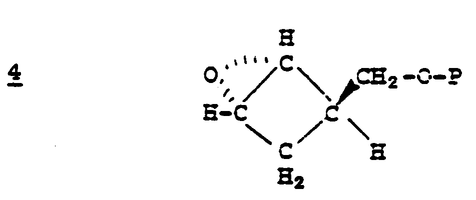 wherein P is a hydroxyl protecting group.

27. A process for preparing compounds of the formula 1 as defined in claim 1, which comprises reacting a compound of the formula 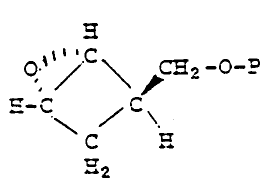 wherein P is a protecting group with a compound of the formula
R₁ H
which is optionally protected and removing the protecting group to yield a compound of formula 1.

28. A process for preparing a compound of the formula 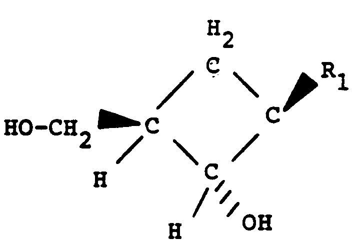 wherein R, is 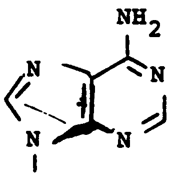 which comprises reacting a compound of the formula 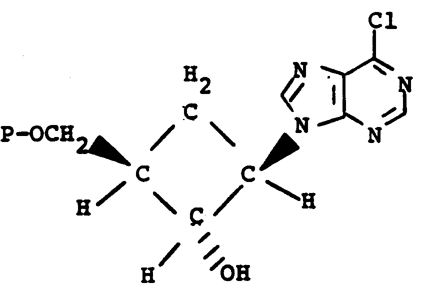 with hot methanolic ammonia followed by removal of the P protecting group.

29. A process for preparing a compound of the formula 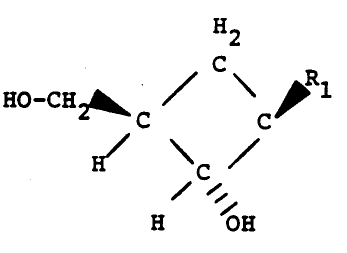 wherein R₁ is 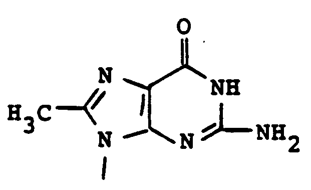 which comprises reacting a compound of the formula 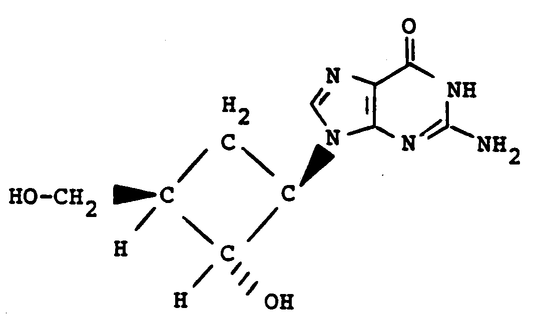 with t-butylhydroperoxide in aqueous sulfuric acid in the presence of ferrous sulfate.

30. A process for preparing a compound of the formula 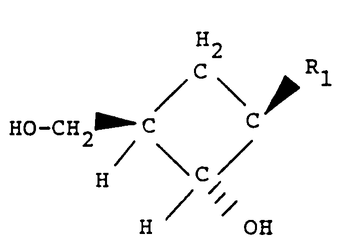 wherein R₁ is 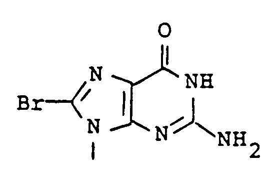 which comprises reacting a compound of the formula 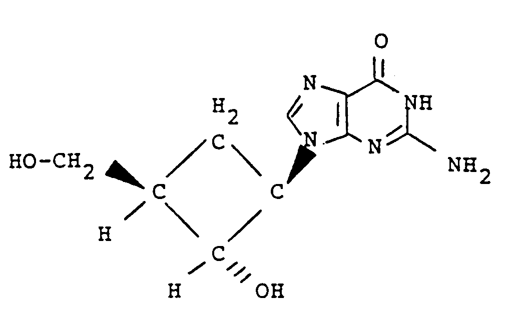 with bromine water.

## Claims (Claims for the following Contracting State(s) : ES)

1. A process for preparing compounds of the formula 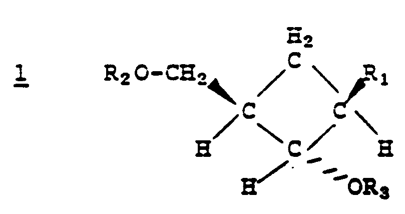 and pharmaceutically acceptable salts thereof wherein R₁ is 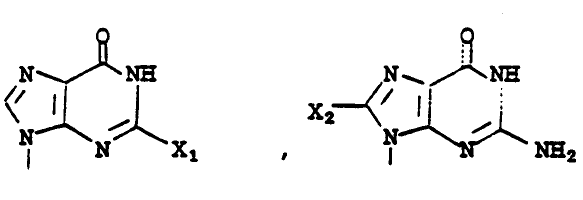 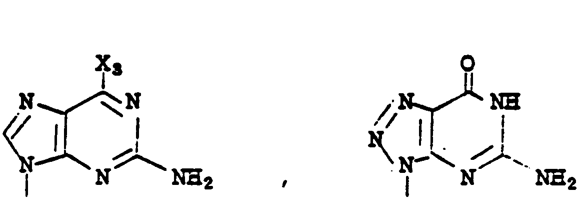 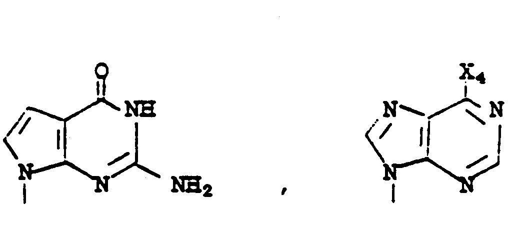 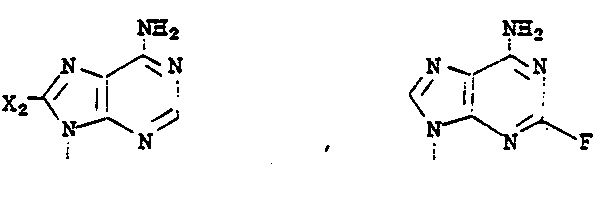 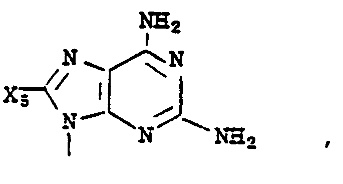 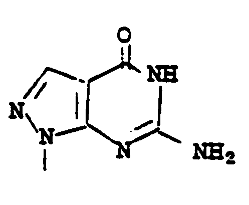 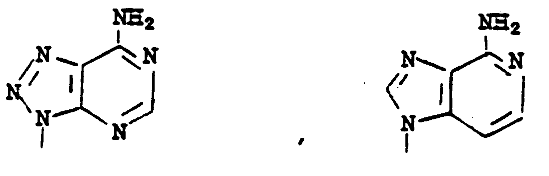 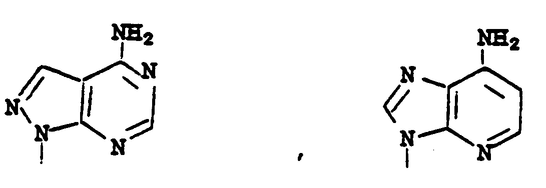 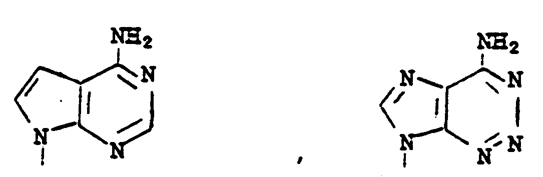 wherein X₁ is hydrogen, amino, 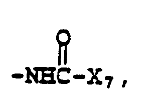 and -N=CHN(X₈)₂
X₂ is methyl, fluoro, chloro, bromo, iodo, hydroxy, or amino,
X₃ is hydrogen, chloro, or O-X₈,
X₄ is amino, 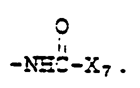 or -N=CHN(X₈)₂,
X₅ is hydrogen, methyl, fluoro, chloro, bromo, iodo, hydroxy, or amino,
X₇ is hydrogen, alkyl, substituted alkyl, or aryl,
X₈ is alkyl,
R₂ and R₃ are independently hydrogen, -P0₃H₂, or 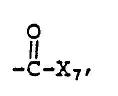 wherein
the term "alkyl" refers to both straight and branched chain groups having 1 to 10 carbons, the term "substituted alkyl" refers to alkyl groups having one or more halogen, amino, azido, hydroxy, cyano, trialkylammonium (wherein each alkyl group has 1 to 6 carbons), alkoxy of 1 to 6 carbons, aryl and carboxy substituents, the term "aryl" refers to phenyl and phenyl substituted with one, two or three alkyl of 1 to 6 carbons, alkoxy of 1 to 6 carbons, halogen, trifluoromethyl, amino, alkylamino, dialkylamino, nitro, cyano, alkanoyloxy of 2 to 11 carbons, carboxy, carbamoyl and hydroxy substituents, which comprises reacting a compound of the formula 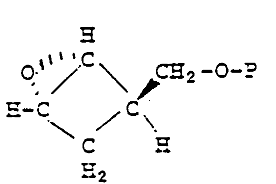 wherein P is a protecting group with a compound of the formula
R₁ H
which is optionally protected and removing the protecting group to yield a compound of formula 1.

2. A process for preparing a compound of the formula 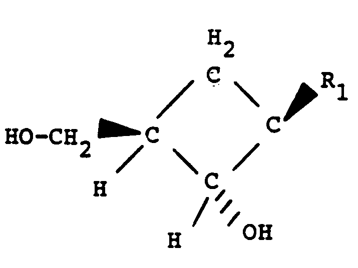 wherein R, is 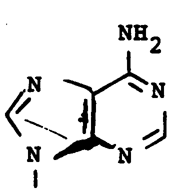 which comprises reacting a compound of the formula 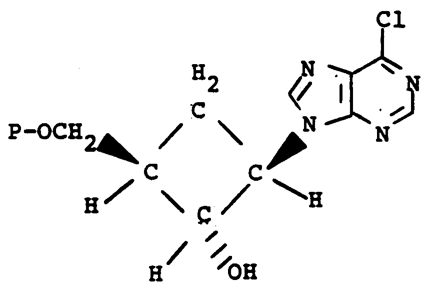 with hot methanolic ammonia followed by removal of the P protecting group.

3. A process for preparing a compound of the formula 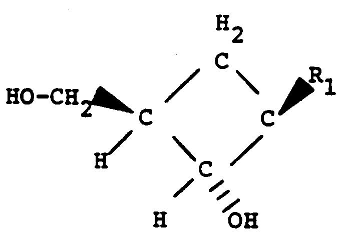 wherein R₁ is 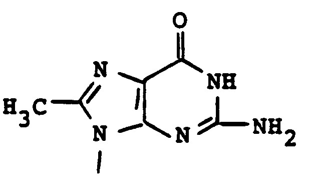 which comprises reacting a compound of the formula 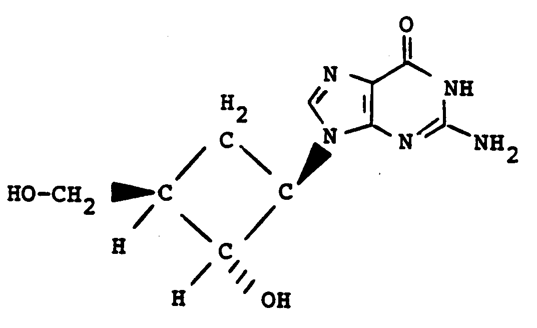 with t-butylhydroperoxide in aqueous sulfuric acid in the presence of ferrous sulfate.

4. A process for preparing a compound of the formula 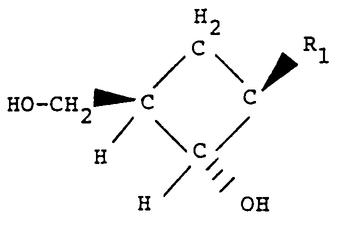 wherein R₁ is 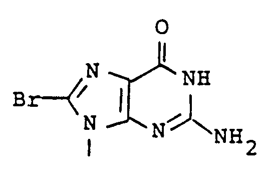 which comprises reacting a compound of the formula 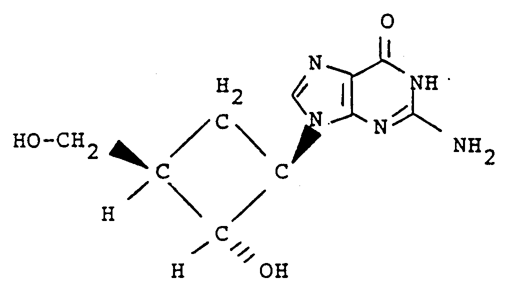 with bromine water.

5. A process in accordance with claim 1 wherein R₁ is 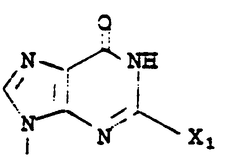 or

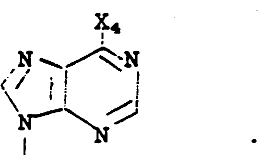

6. A process in accordance with claim 1 wherein R₁ is

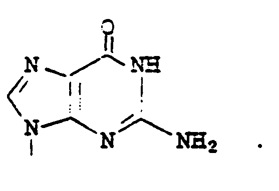

7. A process in accordance with claim 1 or 2 wherein R₁ is

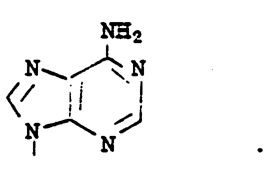

8. A process in accordance with any one or claims 1,6 or 7 wherein R₂ and R₃ are independently hydrogen or

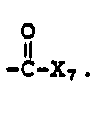

9. A process in accordance with any one of claims 1, 6 or 7 wherein R₂ and R₃ are independently hydrogen or -P0₃H₂.

10. A process in accordance with claim 9 wherein R₂ and R₃ are hydrogen.

11. A process in accordance with claim 1 wherein R₁ is

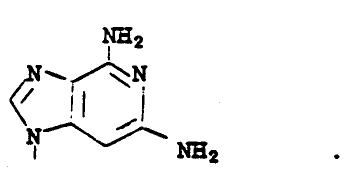

12. A process in accordance with claim 1 wherein R₁ is

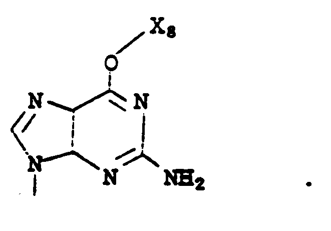

13. A process in accordance with claim 1 wherein R₁ is

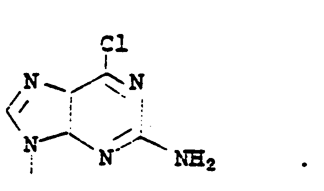

14. A process in accordance with claim 1 wherein R₁ is

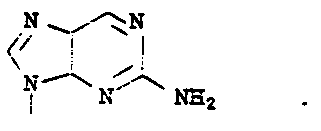

15. A process in accordance with claim 1 wherein R₁ is

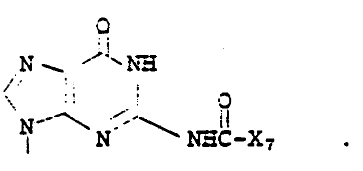

16. A process in accordance with claim 1 wherein R₁ is

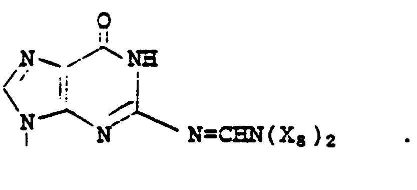

17. A process in accordance with claim 1 wherein R₁ is

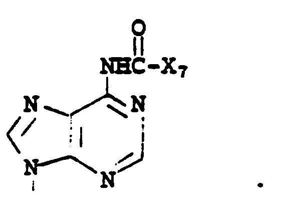

18. A process in accordance with claim 1 wherein R₁ is

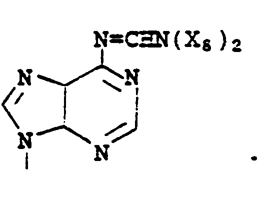

19. The process in accordance with claim 1, wherein the compound prepared is (1a, 2β, 3a)-9-[2-hydroxy-3-(hydroxymethyl)cyclobutyl]guanine.

20. The process in accordance with claim 1, wherein the compound prepared is (1a, 2β, 3a)-3-(6-amino-9H-purin-9-yl)-2-hydroxycyclobutanemethanol.

21. The process in accordance with claim 1, wherein the compound prepared is (1a,2{3,3a)-2-amino-1,9-dihydro-9-[2-hydroxy-3-(hydroxymethyl)cyclobutyl]-8-methyl-6H-purin-6-one.

22. The process in accordance with claim 1, wherein the compound prepared is (1α,2β,3α)-2-amino-8-bromo-1,9-dihydro-9-[2-hydroxy-3-(hydroxymethyl)cyclobutyl]-6H-purin-6-one.

23. The process in accordance with claim 1, wherein the compound prepared is (1α,2β,3α)-2,8-diamino-1,9-dihydro-9-[2-hydroxy-3-(hydroxymethyl)cyclobutyl]-6H-purine-6-one.

24. A method for the preparation of a pharmaceutical composition comprising combining an effective amount of a compound as defined in any one of claims 1 to 23 with a physiologically acceptable carrier.

25. A method according to claim 24 wherein the pharmaceutical composition prepared is an antiviral composition.

26. A method according to claim 25 wherein the antiviral composition prepared is useful in treating a viral infection in a mammalian or avian host.

27. A process for preparing a compound having the formula 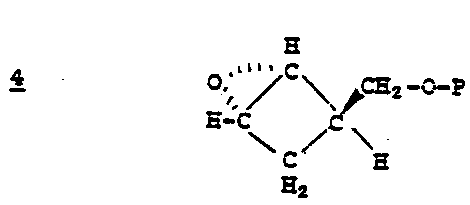 wherein P is a hydroxyl protecting group, comprising epoxidation of a compound of formula

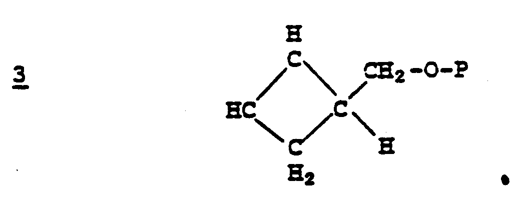

## Claims (Claims for the following Contracting State(s) : GR)

1. A compound having the formula 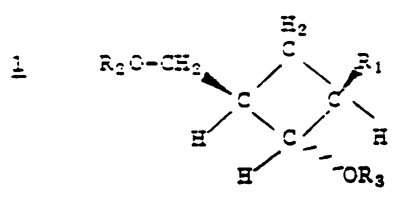 or a pharmaceutically acceptable salt thereof, wherein R₁ is 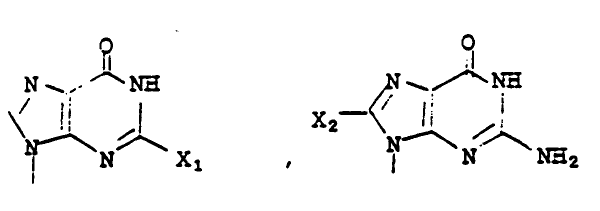 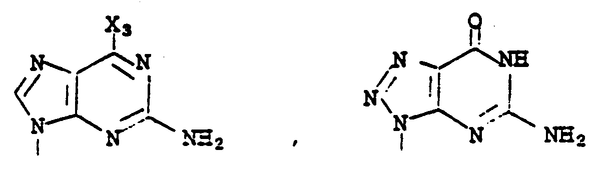 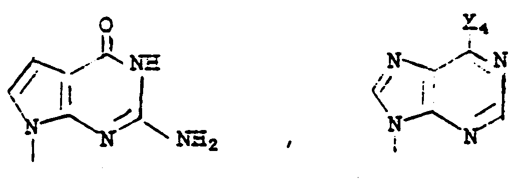 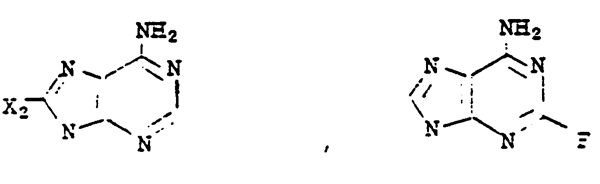 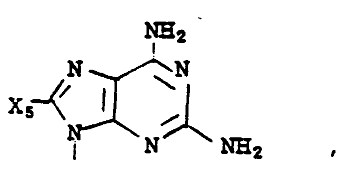 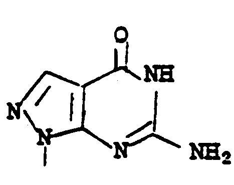 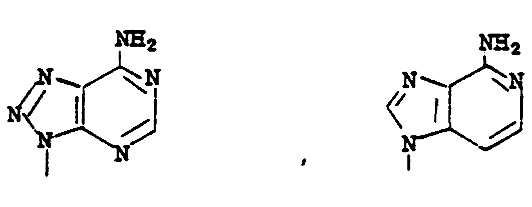 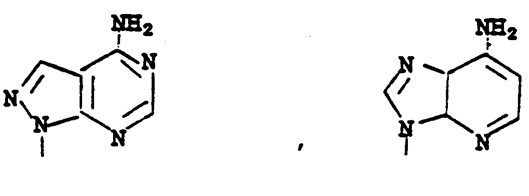 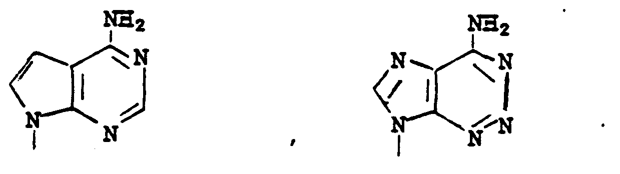 wherein X₁ is hydrogen, amino, 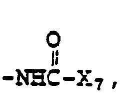 and -N=CHN(X₈)₂
X₂ is methyl, fluoro, chloro, bromo, iodo, hydroxy, or amino,
X₃ is hydrogen, chloro, or O-X₈,
X₄ is amino, 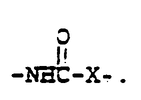 or -N=CHN(X₈)₂,
X₅ is hydrogen, methyl, fluoro, chloro, bromo, iodo, hydroxy, or amino,
X₇ is hydrogen, alkyl, substituted alkyl, or aryl,
X₈ is alkyl,
R₂ and R₃ are independently hydrogen, -P0₃H₂, or 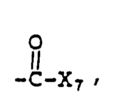 wherein
the term "alkyl" refers to both straight and branched chain groups having 1 to 10 carbons, the term "substituted alkyl" refers to alkyl groups having one or more halogen, amino, azido, hydroxy, cyano, trialkylammonium (wherein each alkyl group has 1 to 6 carbons), alkoxy of 1 to 6 carbons, aryl and carboxy substituents, the term "aryl" refers to phenyl and phenyl substituted with one, two or three alkyl of 1 to 6 carbons, alkoxy of 1 to 6 carbons, halogen, trifluoromethyl, amino, alkylamino, dialkylamino, nitro, cyano, alkanoyloxy of 2 to 11 carbons, carboxy, carbamoyl and hydroxy substituents.

2. A compound in accordance with claim 1 wherein R₁ is 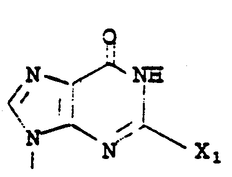 or

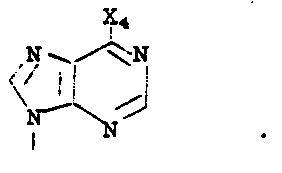

3. A compound in accordance with claim 2 wherein R₁ is

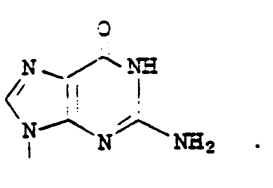

4. A compound in accordance with claim 2 wherein R₁ is

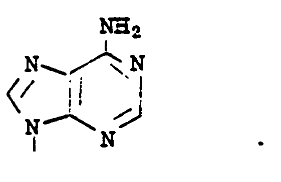

5. A compound in accordance with any one of claims 1, 3 or 4 wherein R₂ and R₃ are independently hydrogen or

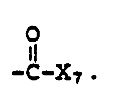

6. A compound in accordance with any one of claims 1, 3 or 4 wherein R₂ and R₃ are independently hydrogen or -P0₃H₂.

7. A compound in accordance with claim 6 wherein R₂ and R₃ are hydrogen.

8. A compound in accordance with claim 1 wherein R₁ is

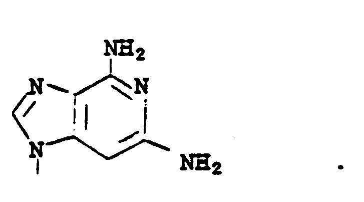

9. A compound in accordance with claim 1 wherein R₁ is

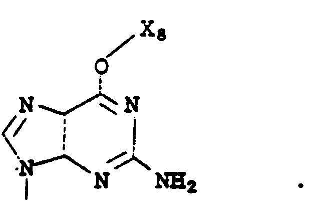

10. A compound in accordance with claim 1 wherein R₁ is

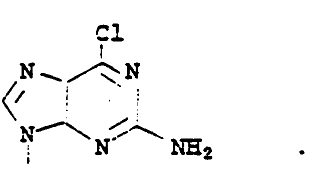

11. A compound in accordance with claim 1 wherein R₁ is

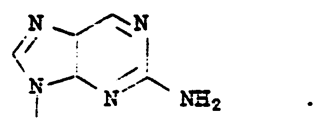

12. A compound in accordance with claim 1 wherein R₁ is

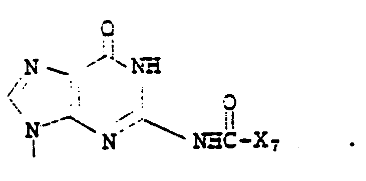

13. A compound in accordance with claim 1 wherein R₁ is

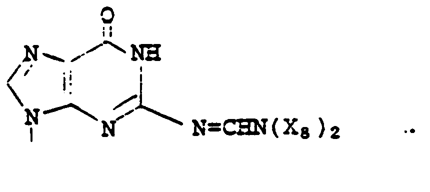

14. A compound in accordance with claim 1 wherein R₁ is

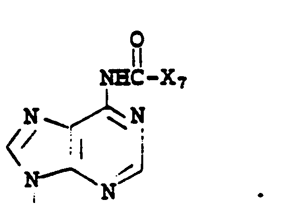

15. A compound in accordance with claim 1 wherein R₁ is

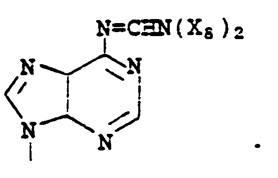

16. The compound in accordance with claim 1, (1α,2β,3α)-9-[2-hydroxy-3-(hydroxymethyl)cyclobutyl]-guanine.

17. The compound in accordance with claim 1, (1α,2β,3α)-3-(6-amino-9H-purin-9-yl)-2-hydrox- ycyclobutanemethanol.

18. The compound in accordance with claim 1, (1α,2β,3α)-2-amino-1,9-dihydro-9-[2-hydroxy-3-(hydroxymethyl)cyclobutyl]-8-methyl-6H-purin-6-one.

19. The compound in accordance with claim 1, (1α,2β,3α)-2-amino-8-bromo-1,9-dihydro-9-[2-hydroxy-3-(hydroxymethyl)cyclobutyl]-6H-purin-6-one.

20. The compound in accordance with claim 1, (1α,2β,3α)-2,8-diamino-1,9-dihydro-9-[2-hydroxy-3-(hydroxymethyl)cyclobutyl]-6H-purine-6-one.

21. A method for the preparation of a pharmaceutical composition comprising combining an effective amount of a compound as defined in any one of claims 1 to 20 with a physiologically acceptable carrier.

22. A method according to claim 21 wherein the pharmaceutical composition prepared is an antiviral composition.

23. A method according to claim 22 wherein the antiviral composition prepared is useful in treating a viral infection in a mammalian or avian host

24. A compound having the formula 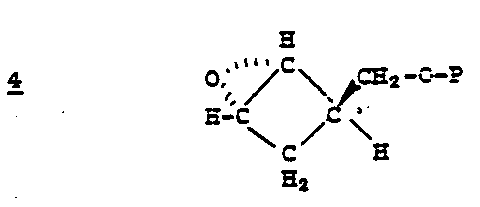 wherein P is a hydroxyl protecting group.

25. A process for preparing compounds of the formula 1 as defined in claim 1, which comprises reacting a compound of the formula 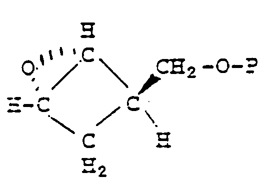 wherein P is a protecting group with a compound of the formula
R₁ H
which is optionally protected and removing the protecting group to yield a compound of formula 1.

26. A process for preparing a compound of the formula 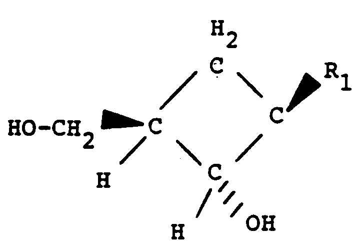 wherein R, is 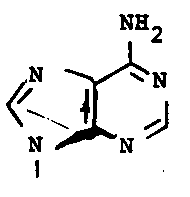 which comprises reacting a compound of the formula 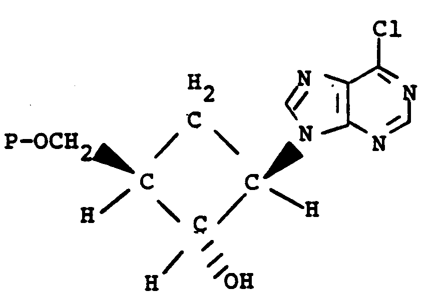 with hot methanolic ammonia followed by removal of the P protecting group.

27. A process for preparing a compound of the formula 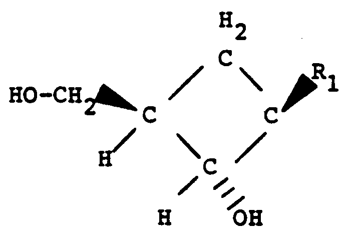 wherein R₁ is 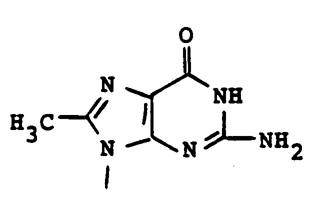 which comprises reacting a compound of the formula 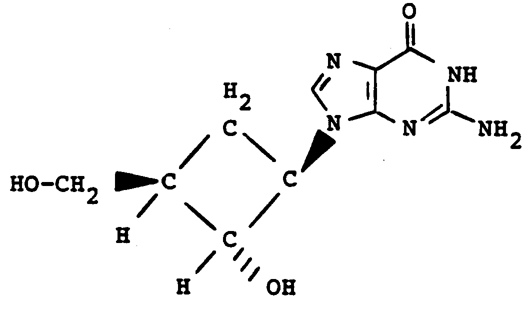 with t-butylhydroperoxide in aqueous sulfuric acid in the presence of ferrous sulfate.

28. A process for preparing a compound of the formula 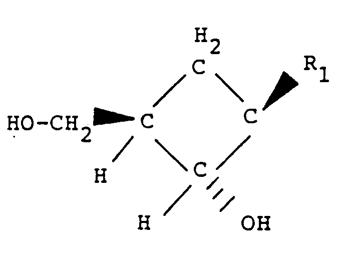 wherein R₁ is 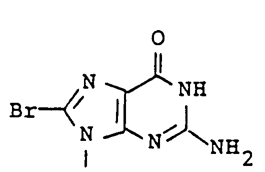 which comprises reacting a compound of the formula 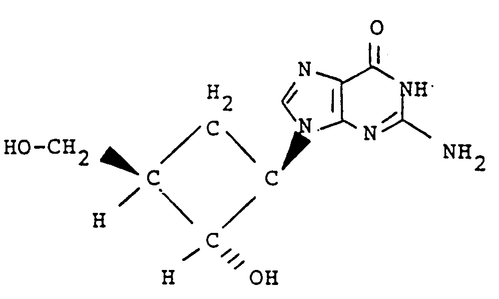 with bromine water.

## Patentansprüche (Patentansprüche für folgende(n) Vertragsstaat(en) : AT, BE, CH, DE, FR, GB, IT, LI, LU, NL, SE)

1. Verbindung der Formel 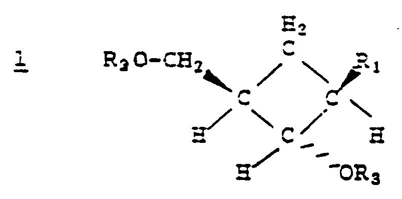 oder ein pharmazeutisch verträgliches Salz davon, wobei R₁ 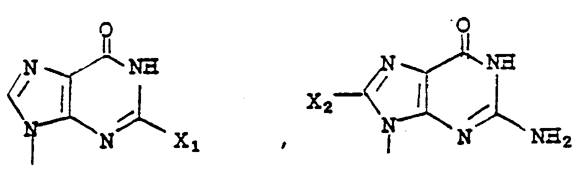 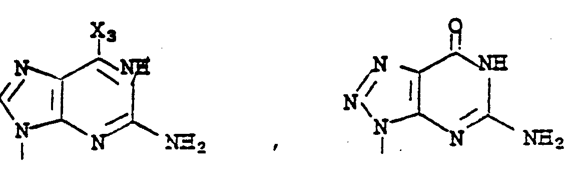 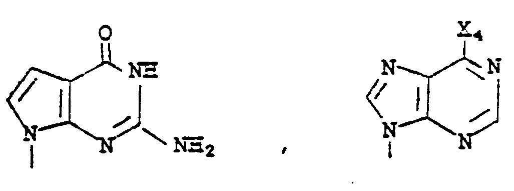 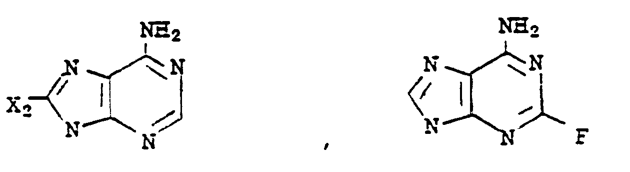 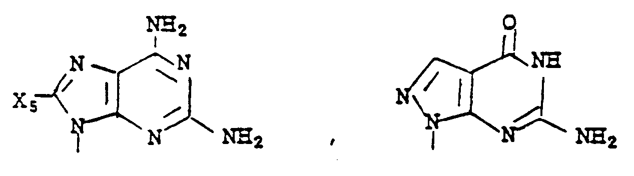 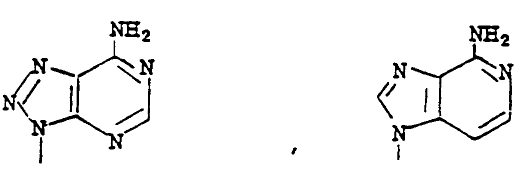 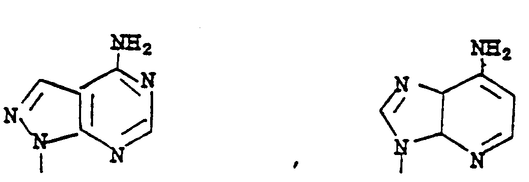 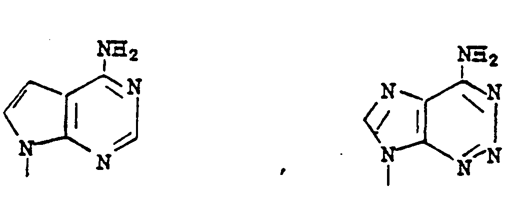 ist, wobei X₁ ein Wasserstoffatom, eine Aminogruppe, 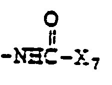 und -N=CHN(X₈)₂ ist,
X₂ eine Methylgruppe, ein Fluor-, Chlor-, Brom-, Jodatom, eine Hydroxyl- oder Aminogruppe ist,
X₃ ein Wasserstoff-, Chloratom oder O-X₈ ist,
X₄ eine Aminogruppe, 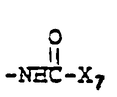 oder -N=CHN(X₈)₂ ist,
X₅ ein Wasserstoffatom, eine Methylgruppe, ein Fluor-, Chlor-, Brom-, Jodatom, eine Hydroxyl- oder Aminogruppe ist,
X₇ ein Wasserstoffatom, Alkyl-, substituierter Alkyl- oder Arylrest ist,
X₈ ein Alkylrest ist,
R₂ und R₃ unabhängig Wasserstoffatome, -P0₃H₂ oder sind, wobei
der Begriff "Alkylrest" sich auf sowohl lineare als auch verzweigte Reste mit 1 bis 10 Kohlenstoffatomen bezieht, der Begriff "substituierter Alkylrest" sich auf Alkylreste mit ein oder mehreren Halogen-, Amino-, Azido-, Hydroxyl-, Cyano-, Trialkylammonium- (wobei jeder Alkylrest 1 bis 6 Kohlenstoffatome aufweist), Alkoxy- mit 1 bis 6 Kohlenstoffatomen, Aryl- und Carboxysubstituenten bezieht; der Begriff "Arylrest" sich auf Phenyl- und substituierte Phenylgruppen mit einem, zwei oder drei Alkyl- mit 1 bis 6 Kohlenstoffatomen, Alkoxymit 1 bis 6 Kohlenstoffatomen, Halogen-, Trifluormethyl-, Amino-, Alkylamino- , Dialxylamino-, Nitro-, Cyano-, Alkanoyloxy- mit 2 bis 11 Kohlenstoffatomen, Carboxy-, Carbamoyl- und Hydroxylsubstituenten bezieht.

2. Verbindung nach Anspruch 1, in der R₁ 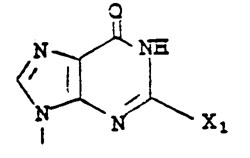 oder 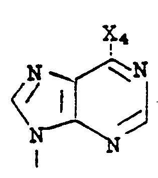 ist.

3. Verbindung nach Anspruch 2, in der R₁ 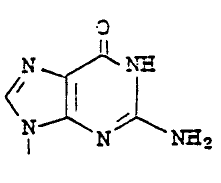 ist.

4. Verbindung nach Anspruch 2, in der R₁ 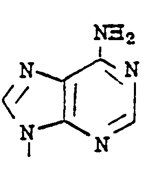 ist.

5. Verbindung nach einem der Ansprüche 1, 3 oder 4, in der R₂ und R₃ unabhängig Wasserstoffatome oder 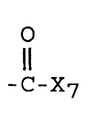 sind.

6. Verbindung nach einem der Ansprüche 1, 3 oder 4, in der R₂ und R₃ unabhängig Wasserstoffatome oder -P0₃H₂ sind.

7. Verbindung nach Anspruch 6, in der R₂ und R₃ Wasserstoffatome sind.

8. Verbindung nach Anspruch 1, in der R₁ 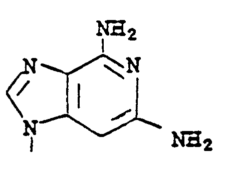 ist.

9. Verbindung nach Anspruch 1, in der R₁ 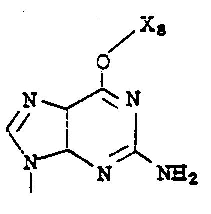 ist.

10. Verbindung nach Anspruch 1, in der R₁ 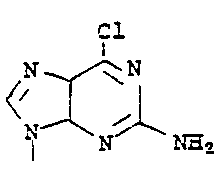 ist.

11. Verbindung nach Anspruch 1, in der R₁ 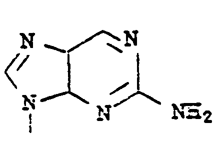 ist.

12. Verbindung nach Anspruch 1, in der R₁ 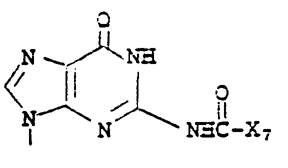 ist.

13. Verbindung nach Anspruch 1, in der R₁ 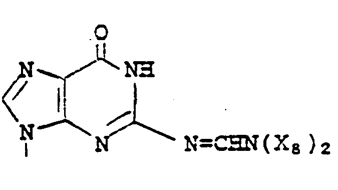 ist.

14. Verbindung nach Anspruch 1, in der R₁ 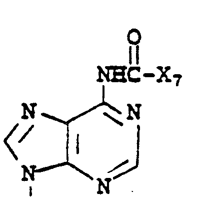 ist.

15. Verbindung nach Anspruch 1, in der R₁ 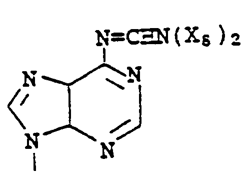 ist.

16. Verbindung nach Anspruch 1, nämlich (1α,2β,3α)-9-[2-Hydroxy-3-(hydroxymethyl)cyclobutyl]guanin.

17. Verbindung nach Anspruch 1, nämlich (1α,2β,3α)-3-(6-Amino-9H-purin-9-yl)-2-hydroxycyclobutanmetha- nol.

18. Verbindung nach Anspruch 1, nämlich (1α,2β,3α)-2-Amino-1,9-dihydro-9-[2-hydroxy-3-(hydroxymethyl)-cyclobutyl]-8-methyl-6H-purin-6-on.

19. Verbindung nach Anspruch 1, nämlich (1α,2β,3α)-2-Amino-8-brom-1,9-dihydro-9-[2-hydroxy-3-(hydroxymethyl)cyclobutyl]-6H-purin-6-on.

20. Verbindung nach Anspruch 1, nämlich (1α,2β,3α)-2,8-Diamino-1,9-dihydro-9-[2-hydroxy-3-(hydroxymethyl)cyclobutyl]-6H-purin-6-on.

21. Verbindung nach einem der Ansprüche 1 bis 20 zur Anwendung als pharmazeutischer Wirkstoff.

22. Verbindung nach einem der Ansprüche 1 bis 20 zur Anwendung bei einem Verfahren zur Behandlung einer Virusinfektion bei einem Säuger- oder Vogelwirt.

23. Arzneimittel, umfassend eine wirksame Menge einer Verbindung nach einem der Ansprüche 1 bis 20 und einen physiologisch verträglichen Träger.

24. Antivirales Mittel, umfassend eine wirksame Menge einer Verbindung nach einem der Ansprüche 1 bis 20 und einen physiologisch verträglichen Träger.

25. Verwendung einer Verbindung nach einemd er Ansprüche 1 bis 20 zur Herstellung eines antiviralen Mittels.

26. Verbindung der Formel 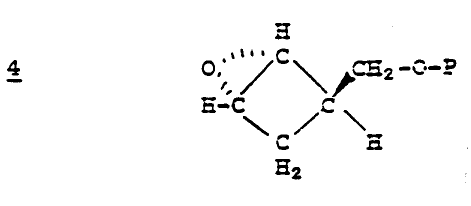 in der P eine Hydroxylschutzgruppe ist.

27. Verfahren zur Herstellung von Verbindungender Formel 1 nach Anspruch 1, umfassend die Umsetzung einer Verbindung der Formel 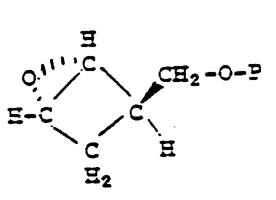 in der P eine Schutzgruppe ist, mit einer Verbindung der Formel
R₁ H
die gegebenenfalls geschützt ist, und Entfernen der Schutzgruppe, wobei eine Verbindung der Formel 1 erhalten wird.

28. Verfahren zur Herstellung einer Verbindung der Formel 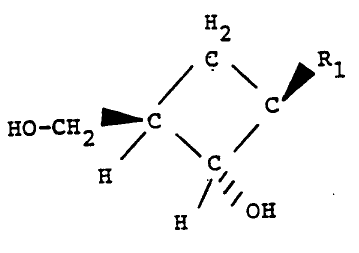 in der R₁ 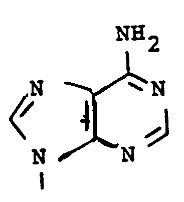 ist, umfassend die Umsetzung einer Verbindung der Formel 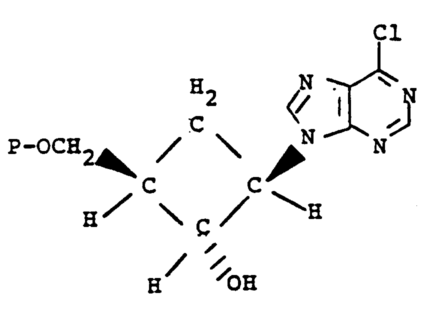 mit heißem methanolischem Ammoniak, anschließend Entfernen der Schutzgruppe P.

29. Verfahren zur Herstellung einer Verbindung der Formel 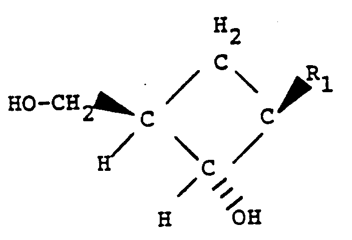 in der R₁ 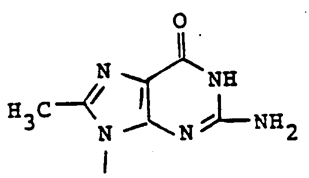 ist, umfassend die Umsetzung einer Verbindung der Formel 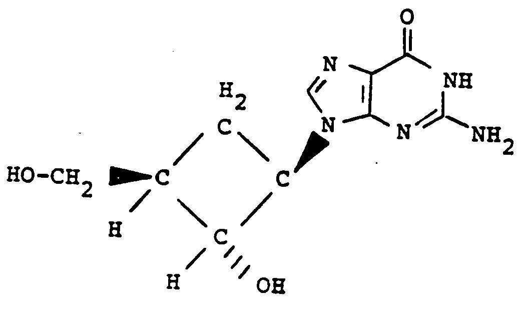 mit tert-Butylhydroperoxid in wäßriger Schwefelsäure in Gegenwart von Eisen(II)-sulfat.

30. Verfahren zur Herstellung einer Verbindung der Formel 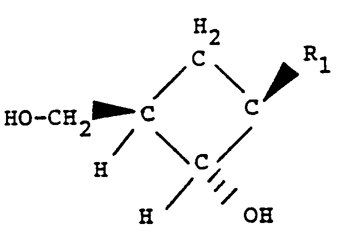 in der R₁ 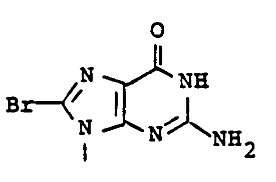 ist, umfassend die Umsetzung einer Verbindung der Formel 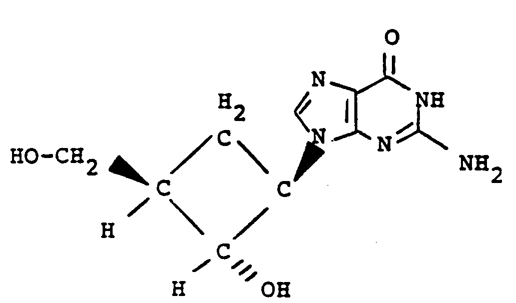 mit Bromwasser.

## Patentansprüche (Patentansprüche für folgende(n) Vertragsstaat(en) : ES)

1. Verfahren zur Herstellung von Verbindungen der Formel 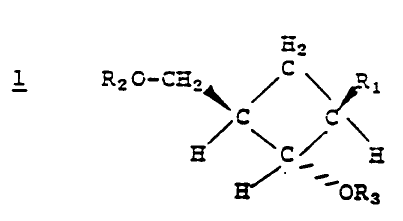 oder pharmazeutisch verträglichen Salzen davon, wobei R₁ 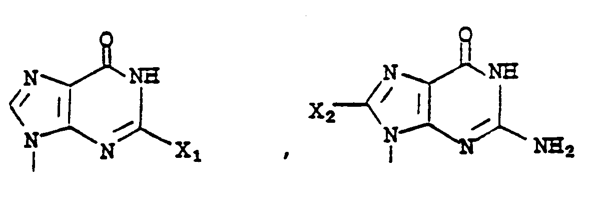 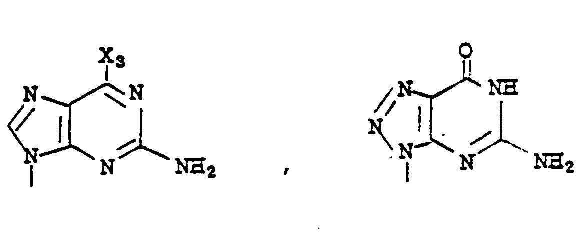 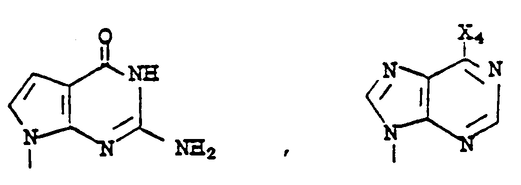 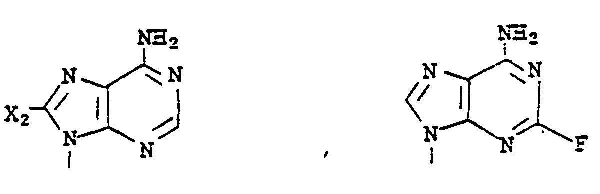 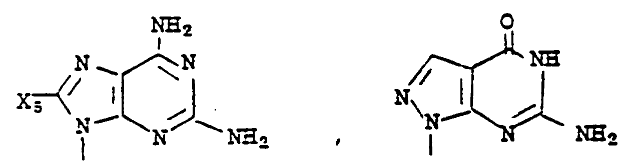 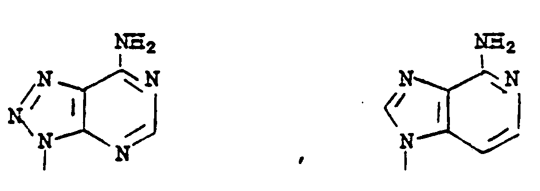 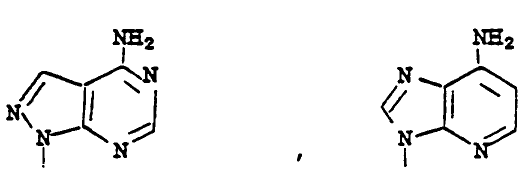 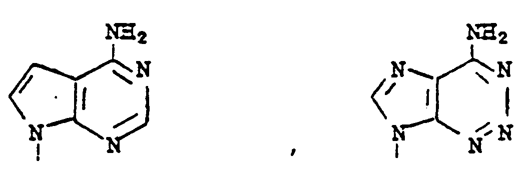 ist, wobei X₁ ein Wasserstoffatom, eine Aminogruppe, 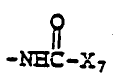 und -N=CHN(X₈)₂ ist,
X₂ eine Methylgruppe, ein Fluor-, Chlor-, Brom-, Jodatom, eine Hydroxyl- oder Aminogruppe ist,
X₃ ein Wasserstoff-, Chloratom oder O-X₈ ist,
X₄ eine Aminogruppe, 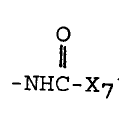 oder -N=CHN(X₈)₂ ist,
X₅ ein Wasserstoffatom, eine Methylgruppe, ein Fluor-, Chlor-, Brom-, Jodatom, eine Hydroxyl- oder Aminogruppe ist,
X₇ ein Wasserstoffatom, Alkyl-, substituierter Alkyl- oder Arylrest ist,
X₈ ein Alkylrest ist,
R₂ und R₃ unabhängig Wasserstoffatome, -P0₃H₂ oder sind, wobei
der Begriff "Alkylrest" sich auf sowohl lineare als auch verzweigte Reste mit 1 bis 10 Kohlenstoffatomen bezieht, der Begriff "substituierter Alkylrest" sich auf Alkylreste mit ein oder mehreren Halogen-, Amino-, Azido-, Hydroxyl-, Cyano-, Trialkylammonium- (wobei jeder Alkylrest 1 bis 6 Kohlenstoffatome aufweist), Alkoxy- mit 1 bis 6 Kohlenstoffatomen, Aryl- und Carboxysubstituenten bezieht; der Begriff "Arylrest" sich auf Phenyl- und substituierte Phenylgruppen mit einem, zwei oder drei Alkyl- mit 1 bis 6 Kohlenstoffatomen, Alkoxy- mit 1 bis 6 Kohlenstoffatomen, Halogen-, Trifluormethyl-, Amino-, Alkylamino-, Dialkylamino-, Nitro-, Cyano-, Alkanoyloxy- mit 2 bis 11 Kohlenstoffatomen, Carboxy, Carbamoyl-und Hydroxylsubstituenten bezieht, umfassend die Umsetzung einer Verbindung der Formel 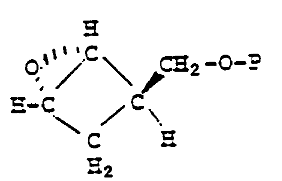 in der P eine Schutzgruppe ist, mit einer Verbindung der Formel
R₁ H
die gegebenenfalls geschützt ist, und Entfernen der Schutzgruppe, wobei eine Verbindung der Formel 1 erhalten wird.

2. Verfahren zur Herstellung einer Verbindung der Formel 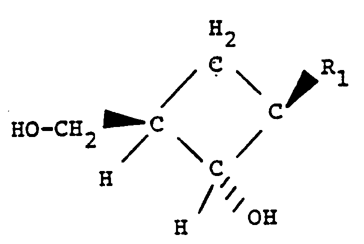 in der R, 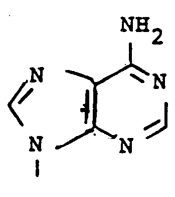 ist, umfassend die Umsetzung einer Verbindung der Formel 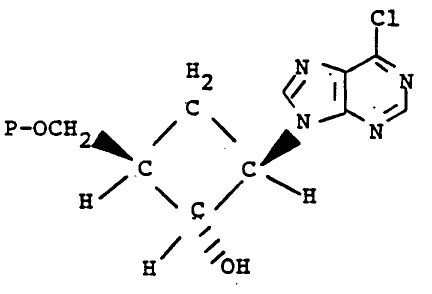 mit heißem methanolischem Ammoniak, anschließend Entfernen der Schutzgruppe P.

3. Verfahren zur Herstellung einer Verbindung der Formel 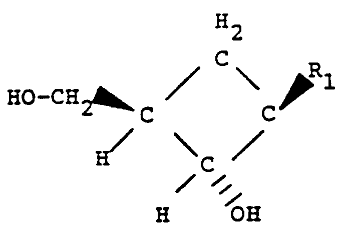 in der R₁ 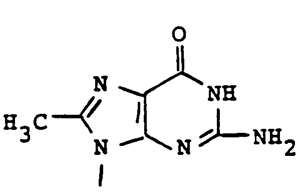 ist, umfassend die Umsetzung einer Verbindung der Formel 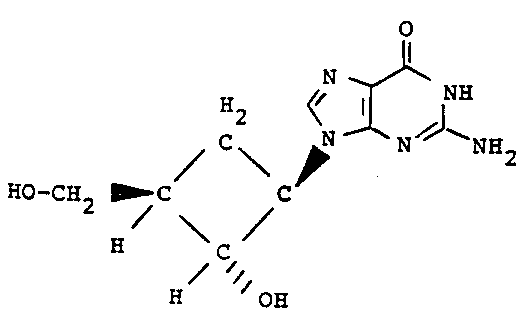 mit tert-Butylhydroperoxid in wäßriger Schwefelsäure in Gegenwart von Eisen(II)-sulfat.

4. Verfahren zur Herstellung einer Verbindung der Formel 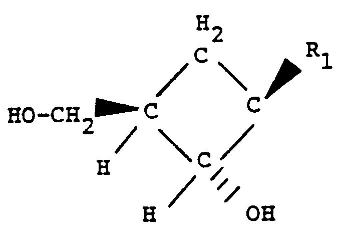 in der R₁ 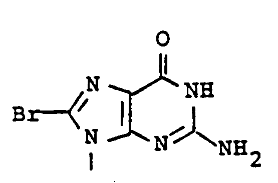 ist, umfassend die Umsetzung einer Verbindung der Formel 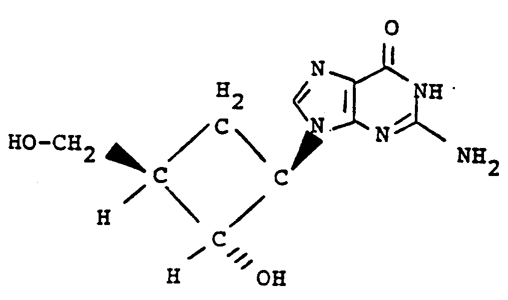 mit Bromwasser.

5. Verfahren nach Anspruch 1, wobei R₁ 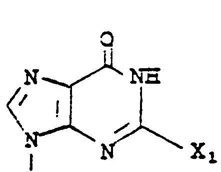 oder 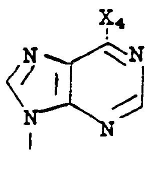 ist.

6. Verfahren nach Anspruch 1, wobei R₁ 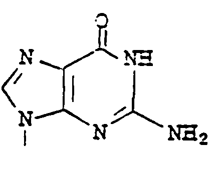 ist.

7. Verfahren nach Anspruch 1 oder 2, wobei R₁ 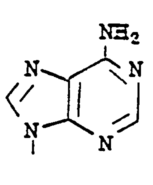 ist.

8. Verfahren nach einem der Ansprüche 1, 6 oder 7, wobei R₂ und R₃ unabhängig Wasserstoffatome oder 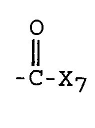 sind.

9. Verfahren nach einem der Ansprüche 1, 6 oder 7, wobei R₂ und R₃ unabhängig Wasserstoffatome oder -PO₃ H₂ sind.

10. Verfahren nach Anspruch 9, wobei R₂ und R₃ Wasserstoffatome sind.

11. Verfahren nach Anspruch 1, wobei R₁ 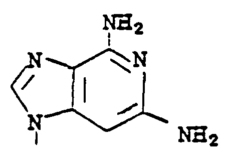 ist.

12. Verfahren nach Anspruch 1, wobei R₁ 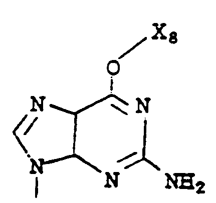 ist.

13. Verfahren nach Anspruch 1, wobei R₁ 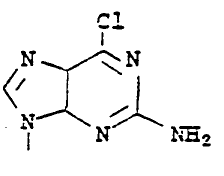 ist.

14. Verfahren nach Anspruch 1, wobei R₁ 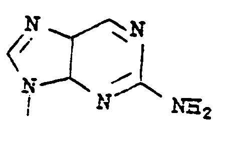 ist.

15. Verfahren nach Anspruch 1, wobei R₁ 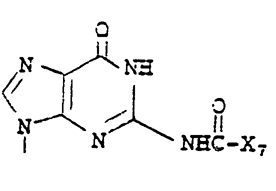 ist.

16. Verfahren nach Anspruch 1, wobei R₁ 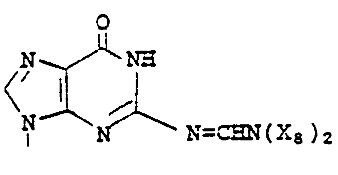 ist.

17. Verfahren nach Anspruch 1, wobei R₁ 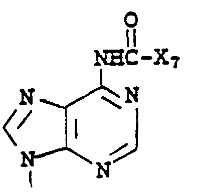 ist.

18. Verfahren nach Anspruch 1, wobei R₁ 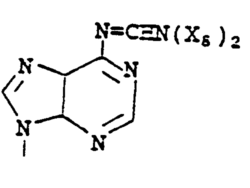 ist.

19. Verfahren nach Anspruch 1, wobei die hergestellte Verbindung (1a,2{3,3a)-9-[2-Hydroxy-3-(hydroxymethyl)cyclobutyl]guanin ist.

20. Verfahren nach Anspruch 1, wobei die hergestellte Verbindung (1α,2β,3α)-3-(6-Amino-9H-purin-9-yl)-2-hydroxycyclobutanmethanol ist.

21. Verfahren nach Anspruch 1, wobei die hergestellte Verbindung (1α,2β,3α)-2-Amino-1,9-dihydro-9-[2-hydroxy-3-(hydroxymethyl)cyclobutyl]-8-methyl-6H-purin-6-on ist.

22. Verfahren nach Anspruch 1, wobei die hergestellte Verbindung (1α,2β,3α)-2-Amino-8-brom-1,9-dihydro-9-[2-hydroxy-3-(hydroxymethyl)cyclobutyl]-6H-purin-6-on ist.

23. Verfahren nach Anspruch 1, wobei die hergestellte Verbindung (1α,2β,3α)-2,8-Diamino-1,9-dihydro-9-[2-hydroxy-3-(hydroxymethyl)cyclobutyl]-6H-purin-6-on ist.

24. Verfahren zur Herstellung eines Arzneimittels, umfassend das Zusammenbringen einer wirksamen Menge einer Verbindung nach einem der Ansprüche 1 bis 23 mit einem physiologisch verträglichen Träger.

25. Verfahren nach Anspruch 24, in dem das hergestellte Arzneimittel ein antivirales Mittel ist.

26. Verfahren nach Anspruch 25, in dem das hergestellte antivirale Mittel zur Behandlung einer Virusinfektion bei einem Säuger- oder Vogelwirt geeignet ist.

27. Verfahren zur Herstellung einer Verbindung der Formel 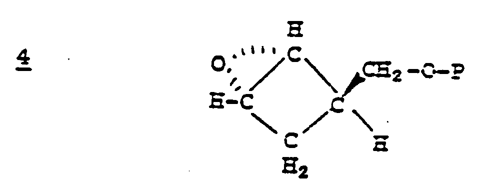 in der P eine Hydroxylschutzgruppe ist, umfassend die Epoxidierung einer Verbindung der Formel

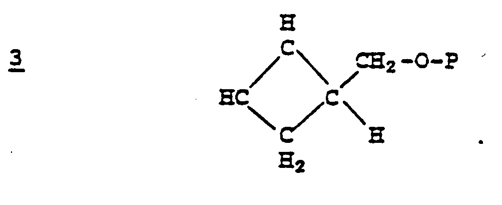

## Patentansprüche (Patentansprüche für folgende(n) Vertragsstaat(en) : GR)

1. Verbindung der Formel 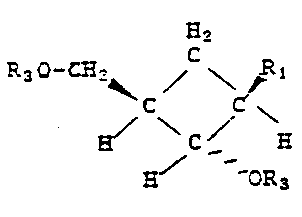 oder ein pharmazeutisch verträgliches Salz davon, wobei R₁ 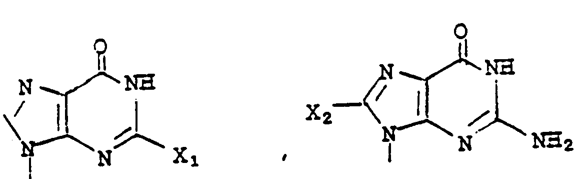 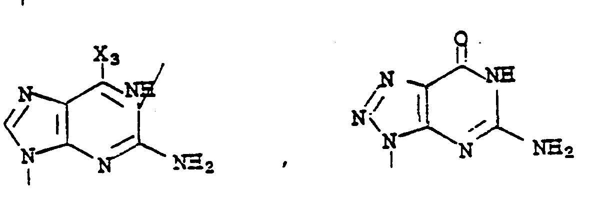 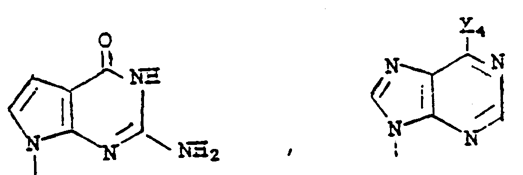 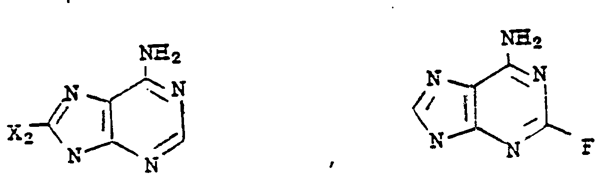 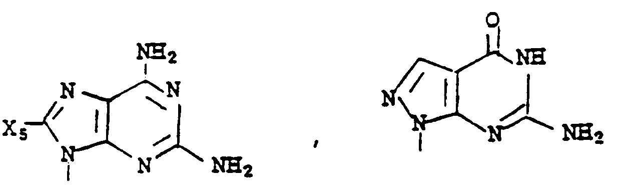 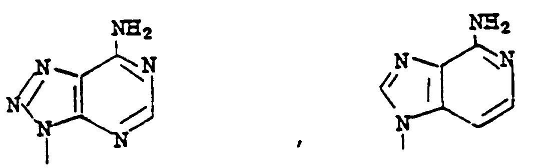 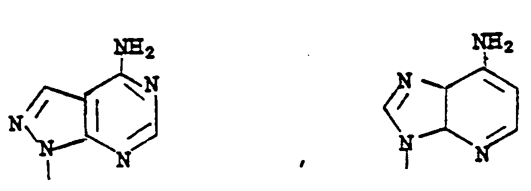 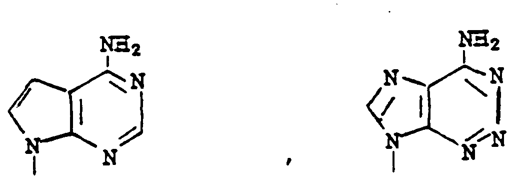 ist, wobei X₁ ein Wasserstoffatom, eine Aminogruppe, 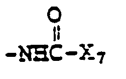 und -N=CHN(X₈)₂ ist,
X₂ eine Methylgruppe, ein Fluor-, Chlor-, Brom-, Jodatom, eine Hydroxyl- oder Aminogruppe ist,
X₃ ein Wasserstoff-, Chloratom oder O-X₈ ist,
X₄ eine Aminogruppe, 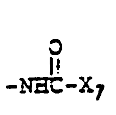 oder -N=CHN(X₈)₂ ist,
X₅ ein Wasserstoffatom, eine Methylgruppe, ein Fluor-, Chlor-, Brom-, Jodatom, eine Hydroxyl- oder Amino-, Methyl-, Trifluormethyl-, Ethyl-, 2-Fluorethylgruppe ist,
X₇ ein Wasserstoffatom, Alkyl-, substituierter Alkyl- oder Arylrest ist,
X₈ ein Alkylrest ist,
R₂ und R₃ unabhängig Wasserstoffatome, -P0₃H₂ oder sind, wobei
der Begriff "Alkylrest" sich auf sowohl lineare als auch verzweigte Reste mit 1 bis 10 Kohlenstoffatomen bezieht, der Begriff "substituierter Alkylrest" sich auf Alkylreste mit ein oder mehreren Halogen-, Amino-, Azido-, Hydroxyl-, Cyano-, Trialkylammonium- (wobei jeder Alkylrest 1 bis 6 Kohlenstoffatome aufweist), Alkoxy- mit 1 bis 6 Kohlenstoffatomen, Aryl- und Carboxysubstituenten bezieht; der Begriff "Arylrest" sich auf Phenyl- und substituierte Phenylgruppen mit einem, zwei oder drei Alkyl- mit 1 bis 6 Kohlenstoffatomen, Alkoxymit 1 bis 6 Kohlenstoffatomen, Halogen-, Trifluormethyl-, Amino-, Alkylamino- , Dialkylamino-, Nitro-, Cyano-, Alkanoyloxy- mit 2 bis 11 Kohlenstoffatomen, Carboxy-, Carbamoyl- und Hydroxylsubstituenten bezieht.

2. Verbindung nach Anspruch 1, in der R₁ oder 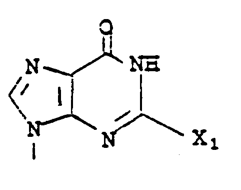 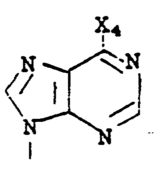 ist.

3. Verbindung nach Anspruch 2, in der R₁ 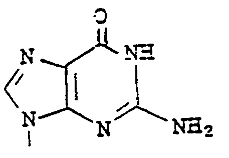 ist.

4. Verbindung nach Anspruch 2, in der R₁ 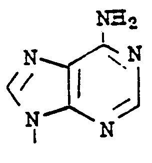 ist.

5. Verbindung nach einem der Ansprüche 1, 3 oder 4, in der R₂ und R₃ unabhängig Wasserstoffatome oder 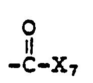 sind.

6. Verbindung nach einem der Ansprüche 1, 3 oder 4, in der R₂ und R₃ unabhängig Wasserstoffatome oder -P0₃H₂ sind.

7. Verbindung nach Anspruch 6, in der R₂ und R₃ Wasserstoffatome sind.

8. Verbindung nach Anspruch 1, in der R₁ 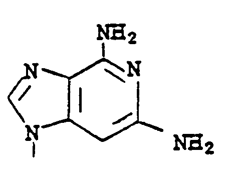 ist.

9. Verbindung nach Anspruch 1, in der R₁ 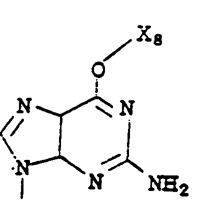 ist.

10. Verbindung nach Anspruch 1, in der R₁ 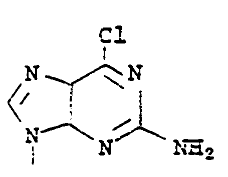 ist.

11. Verbindung nach Anspruch 1, in der R₁ 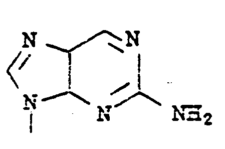 ist.

12. Verbindung nach Anspruch 1, in der R₁ 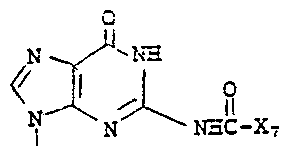 ist.

13. Verbindung nach Anspruch 1, in der R₁ 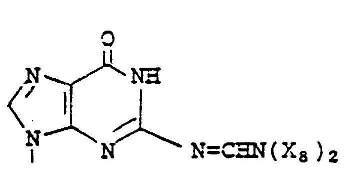 ist.

14. Verbindung nach Anspruch 1, in der R₁ 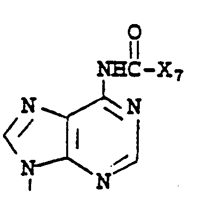 ist.

15. Verbindung nach Anspruch 1, in der R₁ 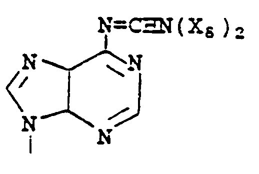 ist.

16. Verbindung nach Anspruch 1, nämlich (1α,2β,3α)-9-[2-Hydroxy-3-(hydroxymethyl)cyclobutyl]guanin.

17. Verbindung nach Anspruch 1, nämlich (1α,2β,3α)-3-(6-Amino-9H-purin-9-yl)-2-hydroxycyclobutanmetha- nol.

18. Verbindung nach Anspruch 1, nämlich (1α,2β,3α)-2-Amino-1,9-dihydro-9-[2-hydroxy-3-(hydroxymethyl)-cyclobutyl]-8-methyl-6H-purin-6-on.

19. Verbindung nach Anspruch 1, nämlich (1α,2β,3α)-2-Amino-8-brom-1,9-dihydro-9-[2-hydroxy-3-(hydroxymethyl)cyclobutyl]-6H-purin-6-on.

20. Verbindung nach Anspruch 1, nämlich (1α,2β,3α)-2,8-Diamino-1,9-dihydro-9-[2-hydroxy-3-(hydroxymethyl)cyclobutyl]-6H-purin-6-on.

21. Verfahren zur Herstellung eines Arzneimittels, umfassend das Zusammenbringen einer wirksamen Menge einer Verbindung nach einem der Ansprüche 1 bis 20 mit einem physiologisch verträglichen Träger.

22. Verfahren nach Anspruch 21, in dem das hergestellte Arzneimittel ein antivirales Mittel ist.

23. Verfahren nach Anspruch 22, in dem das hergestellte antivirale Mittel zur Behandlung einer Virusinfektion bei einem Säuger- oder Vogelwirt geeignet ist.

24. Verbindung der Formel 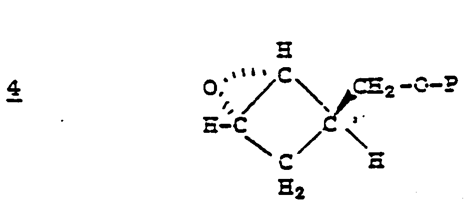 in der P eine Hydroxylschutzgruppe ist.

25. Verfahren zur Herstellung von Verbindungen der Formel 1 nach Anspruch 1, umfassend die Umsetzung einer Verbindung der Formel 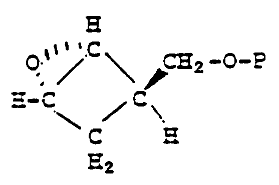 in der P eine Schutzgruppe ist, mit einer Verbindung der Formel
Rᵢ H
die gegebenenfalls geschützt ist, und Entfernen der Schutzgruppe, wobei eine Verbindung der Formel 1 erhalten wird.

26. Verfahren zur Herstellung einer Verbindung der Formel 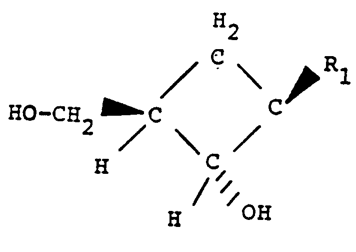 in der R₁ 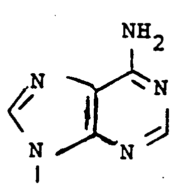 ist, umfassend die Umsetzung einer Verbindung der Formel 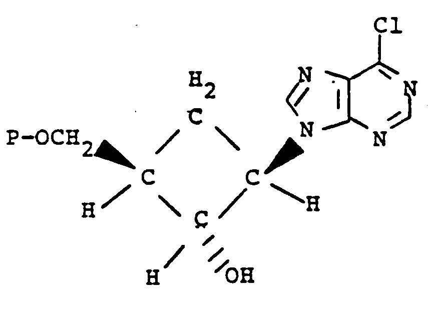 mit heißem methanolischem Ammoniak, anschließend Entfernen der Schutzgruppe P.

27. Verfahren zur Herstellung einer Verbindung der Formel 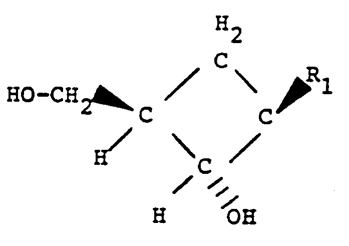 in der R₁ 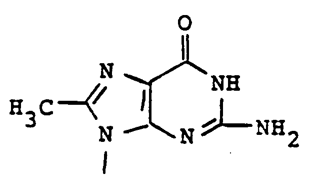 ist, umfassend die Umsetzung einer Verbindung der Formel 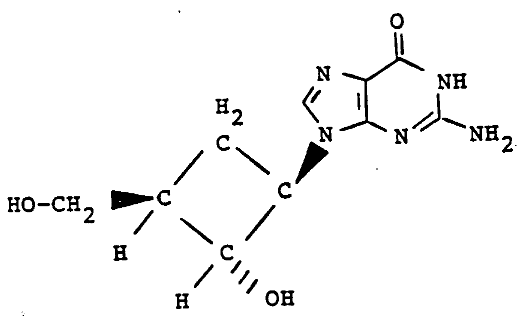 mit tert-Butylhydroperoxid in wäßriger Schwefelsäure in Gegenwart von Eisen(II)-sulfat.

28. Verfahren zur Herstellung einer Verbindung der Formel 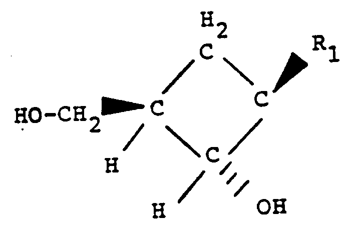 in der R₁ 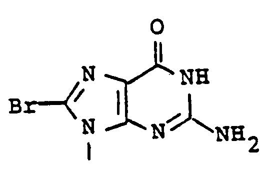 ist, umfassend die Umsetzung einer Verbindung der Formel 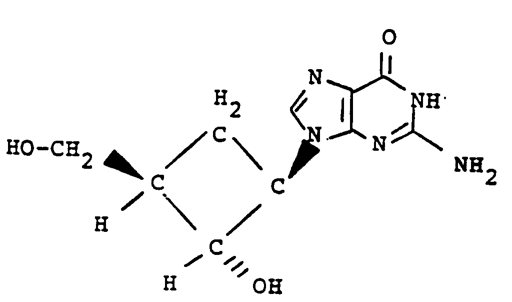 mit Bromwasser.

## Revendications (Revendications pour l'(les) Etat(s) contractant(s) suivant(s) : suivants : AT, BE, CH, DE, FR, GB, IT, LI, LU, NL, SE)

1. Composé ayant pour formule 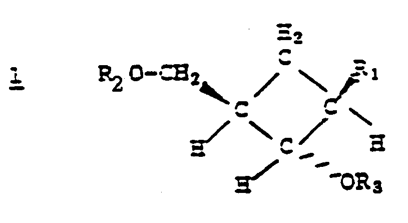 ou sel pharmaceutiquement acceptable de celui-ci, formule dans laquelle R₁ est 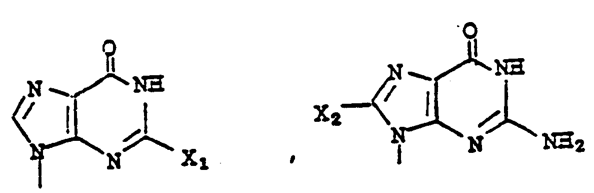 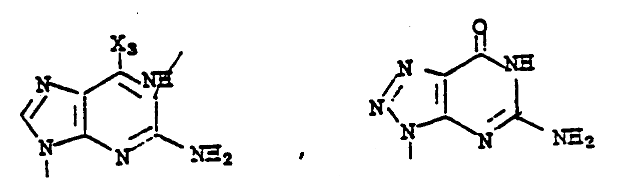 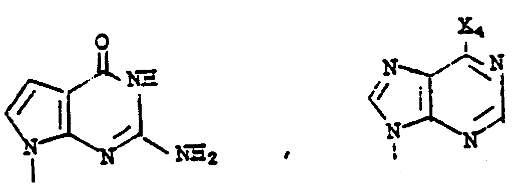 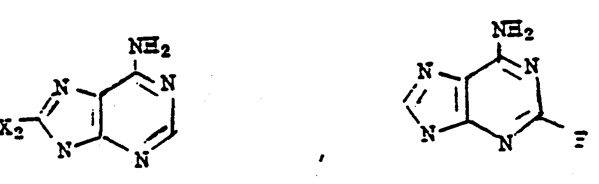 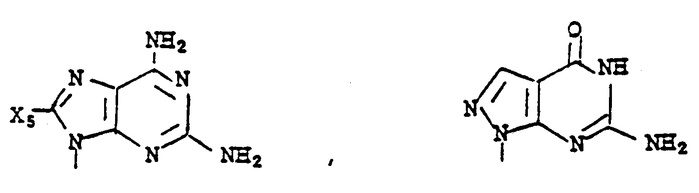 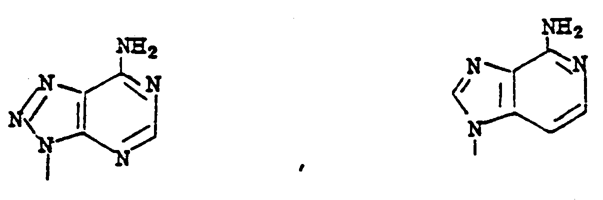 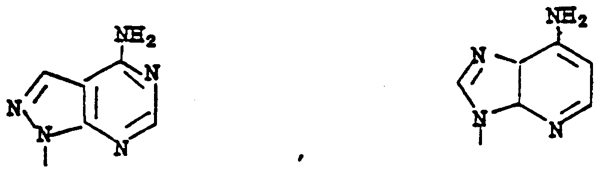 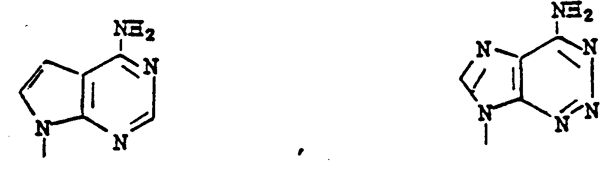 où X₁ est l'hydrogène ou un groupe amine, 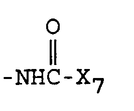 ou -N = CHN(X₈)₂,
X₂ est un groupe méthyle, un atome de fluor, de chlore, de brome ou d'iode, ou un groupe hydroxy ou amine,
X₃ est un atome d'hydrogène ou de chlore, ou O-X₈,
X₄ est un groupe amine, 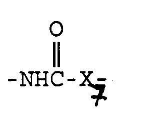 ou -N=CHN(X₈)₂,
X₅ est l'hydrogène, le groupe méthyle, un atome de fluor, de chlore, de brome ou d'iode, ou un groupe hydroxy ou amine,
X₇ est l'hydrogène ou un groupe alkyle, alkyle substitué ou aryle,
X₈ est un groupe alkyle,
R₂ et R₃ sont indépendamment l'hydrogène ou un groupe -P0₃H₂ ou 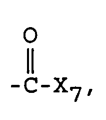 où
le terme "alkyle" désigne un groupe à chaîne droite ou ramifiée ayant de 1 à 10 atomes de carbone, le terme "alkyle substitué" désigne un groupe alkyle ayant un ou plusieurs substituants halogène, amine, azide, hydroxy, cyano, trialkylammonium (où chaque groupe alkyle a de 1 à 6 atomes de carbone), alcoxy de 1 à 6 atomes de carbone , aryle ou carboxy, le terme "aryle" désigne un groupe phényle éventuellement substitué par un, deux ou trois substituants alkyle de 1 à 6 atomes de carbone, alcoxy de 1 à 6 atomes de carbone, halogène, trifluorométhyle, amine, alkylamine, dialkylamine, nitro, cyano, alcanoyloxy de 2 à 11 atomes de carbone, carboxy, carbamoyle ou hydroxy.

2. Composé selon la revendication 1, dans lequel R₁ est 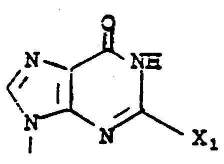 ou

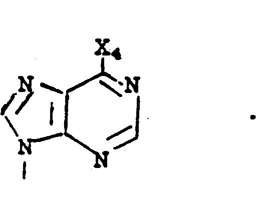

3. Composé selon la revendication 2, dans lequel R₁ est

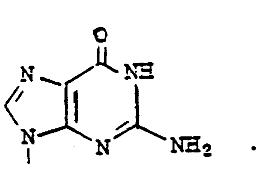

4. Composé selon la revendication 2, dans lequel R₁ est

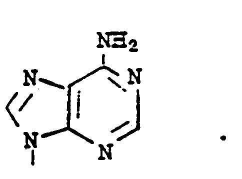

5. Composé selon l'une quelconque des revendications 1, 3 ou 4, dans lequel R₂ et R₃ sont indépendamment l'hydrogène ou

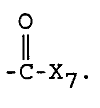

6. Composé selon l'une quelconque des revendications 1, 3 ou 4, dans lequel R₂ et R₃ sont indépendamment l'hydrogène ou -P0₃H₂.

7. Composé selon la revendication 6, dans lequel R₂ et R₃ sont l'hydrogène.

8. Composé selon la revendication 1, dans lequel R₁ est

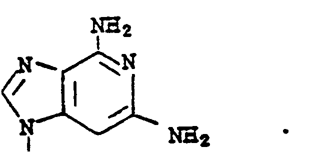

9. Composé selon la revendication 1, dans lequel R₁ est

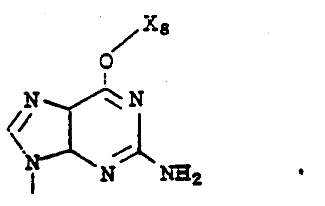

10. Composé selon la revendication 1, dans lequel R₁ est

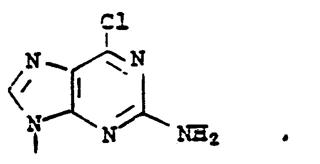

11. Composé selon la revendication 1, dans lequel R₁ est

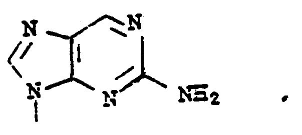

12. Composé selon la revendication 1, dans lequel Ri est

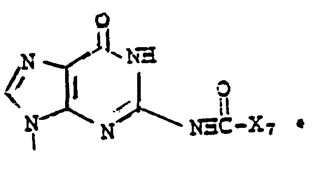

13. Composé selon la revendication 1, dans lequel R₁ est

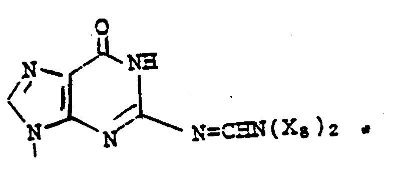

14. Composé selon la revendication 1, dans lequel R₁ est

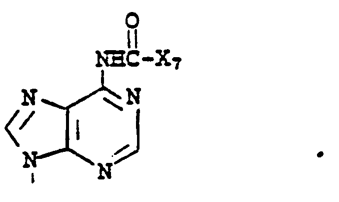

15. Composé selon la revendication 1, dans lequel R₁ est

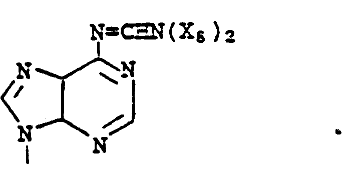

16. Composé selon la revendication 1, qui est la (1α,2β,3α)-9-[2-hydroxy-3-(hydroxyméthyl)cyclobutyl]-guanine.

17. Composé selon la revendication 1, qui est le (1α,2β,3α)-3-(6-amino-9H-purin-9-yl)-2-hydroxycyclobuta- neméthanol.

18. Composé selon la revendication 1, qui est la (1α,2β,3α)-2-amino-1,9-dihydro-9-[2-hydroxy-3-(hydroxy- méthyl)cyclobutyl]-8-méthyl-6H-purin-6-one.

19. Composé selon la revendication 1, qui est la (1α,2β,3α)-2-amino-8-bromo-1,9-dihydro-9-[2-hydroxy-3-(hydroxyméthyl)cyclobutyl]-6H-purin-6-one.

20. Composé selon la revendication 1, qui est (1α,2β,3α)-2,8-diamino-1,9-dihydro-9-[2-hydroxy-3-(hydroxy- méthyl)cyclobutyl]-6H-purin-6-one.

21. Composé selon l'une quelconque des revendications 1 à 20, destiné à être utilisé comme agent à action pharmaceutique.

22. Composé selon l'une quelconque des revendications 1 à 20, destiné à être utilisé dans une méthode de traitement d'une infection virale chez un hôte mammifère ou avien.

23. Composition pharmaceutique comprenant une quantité efficace d'un composé tel qu'il est défini dans l'une quelconque des revendications 1 à 20 et un porteur physiologiquement acceptable.

24. Composition antivirale comprenant une quantité efficace d'un composé tel qu'il est défini dans l'une quelconque des revendications 1 à 20 et un porteur physiologiquement acceptable.

25. Utilisation d'un composé tel qu'il est défini dans l'une quelconque des revendications 1 à 20 pour préparer une composition antivirale.

26. Composé ayant pour formule 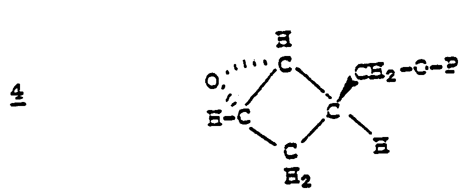 dans laquelle P est un groupe protecteur de la fonction hydroxyle.

27. Procédé de préparation de composés de formule 1 tels qu'ils sont définis dans la revendication 1, qui consiste à faire réagir un composé de formule 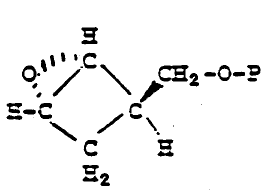 dans laquelle P est un groupe protecteur, avec un composé de formule
Rᵢ H
qui est facultativement protégé, et à éliminer le groupe protecteur pour obtenir un composé de formule 1.

28. Procédé de préparation d'un composé de formule 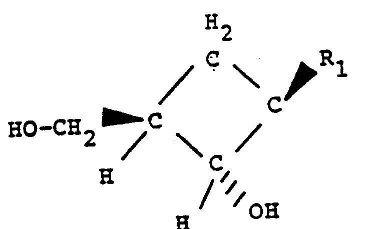 dans laquelle R₁ est 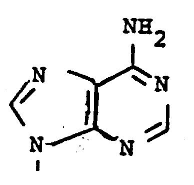 qui consiste à faire réagir un composé de formule 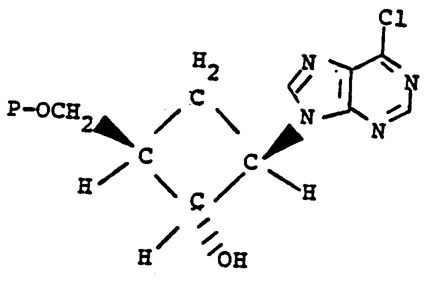 avec de l'ammoniaque méthanolique chaude, puis à éliminer le groupe protecteur P.

29. Procédé de préparation d'un composé de formule 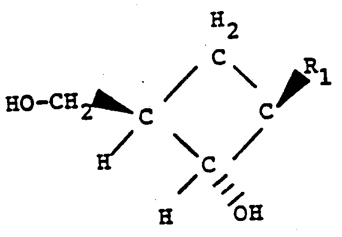 dans laquelle R₁ est 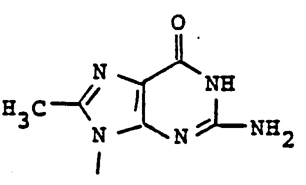 qui consiste à faire réagir un composé de formule 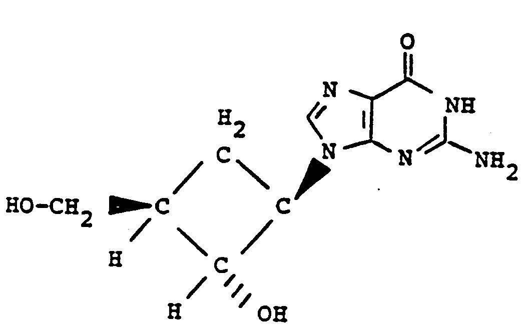 avec de l'hydroperoxyde de t-butyle dans de l'acide sulfurique aqueux, en présence de sulfate ferreux.

30. Procédé de préparation d'un composé de formule 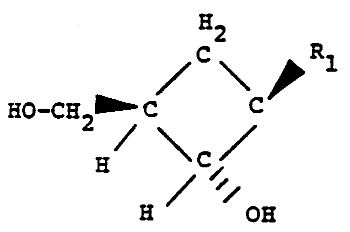 dans laquelle R₁ est 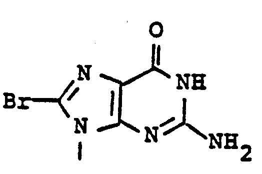 qui consiste à faire réagir un composé de formule 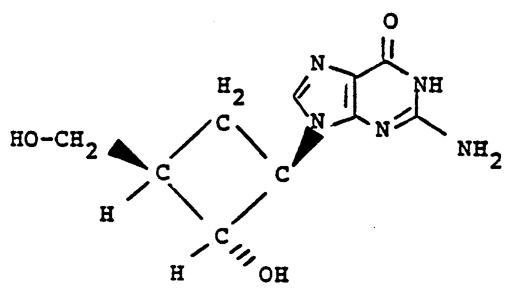 avec de l'eau de brome.

## Revendications (Revendications pour l'(les) Etat(s) contractant(s) suivant(s) : suivant : ES)

1. Procédé de préparation de composés de formule 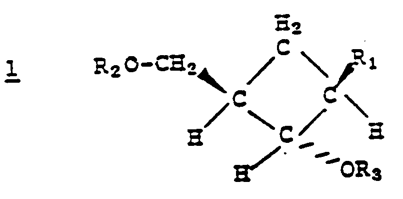 et de leurs sels pharmaceutiquement acceptables, formule dans laquelle R₁ est 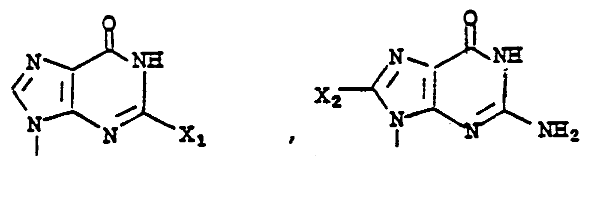 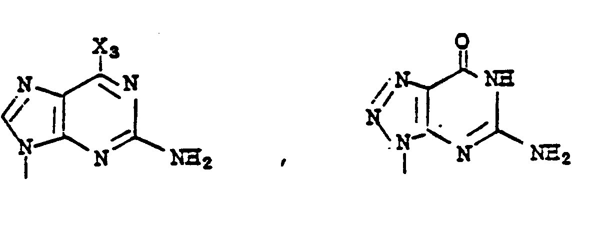 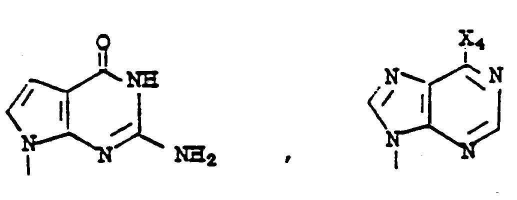 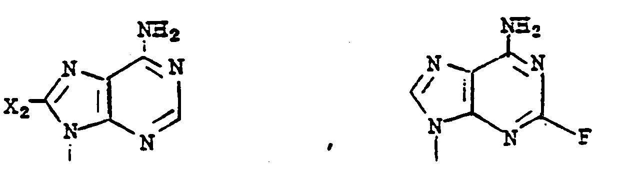 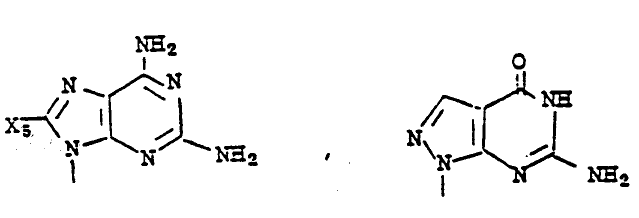 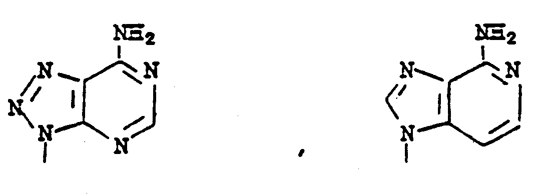 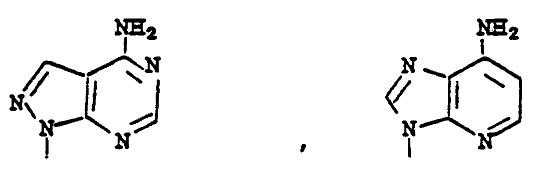 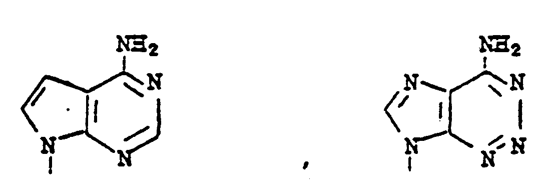 où X₁ est l'hydrogène ou un groupe amine, 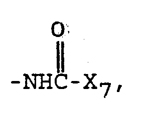 ou -N = CHN(X₈)₂,
X₂ est le groupe méthyle, un atome de fluor, de chlore, de brome ou d'iode, ou un groupement hydroxy ou amine,
X₃ est l'hydrogène, le chlore, ou O-X₈,
X₄ est un groupe amine, 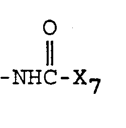 ou -N = CHN(X₈)₂,
X₅ est l'hydrogène, le groupe méthyle, un atome de fluor, de chlore, de brome ou d'iode, ou un groupe hydroxy ou amine,
X₇ est l'hydrogène ou un groupe alkyle, alkyle substitué ou aryle,
X₈ est un groupe alkyle,
R₂ et R₃ sont indépendamment l'hydrogène ou un groupe -P0₃H₂ ou 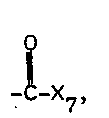 où
le terme "alkyle" désigne un groupe à chaîne droite ou ramifiée ayant de 1 à 10 atomes de carbone, le terme "alkyle substitué" désigne un groupe alkyle ayant un ou plusieurs substituants halogène, amine, azide, hydroxy, cyano, trialkylammonium (où chaque groupe alkyle a de 1 à 6 atomes de carbone), alcoxy de 1 à 6 atomes de carbone, aryle ou carboxy, le terme "aryle" désigne un groupe phényle éventuellement substitué par un, deux ou trois substituants alkyle de 1 à 6 atomes de carbone, alcoxy de 1 à 6 atomes de carbone, halogène, trifluorométhyle, amine, alkylamine, dialkylamine, nitro, cyano, alcanoyloxy de 2 à 11 atomes de carbone, carboxy, carbamoyle ou hydroxy, qui consiste à faire réagir un composé de formule 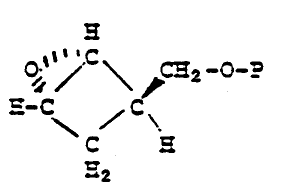 dans laquelle P est un groupe protecteur, avec un composé de formule
R₁ H
qui est facultativement protégé, et à éliminer le groupe protecteur pour obtenir un composé de formule 1.

Procédé de préparation d'un composé de formule 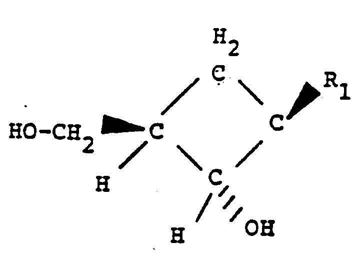 dans laquelle Ri est 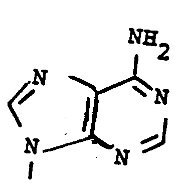 qui consiste à faire réagir un composé de formule 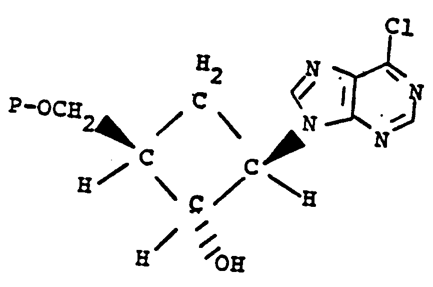 avec de l'ammoniaque méthanolique chaude, puis à éliminer le groupe protecteur P.

3. Procédé de préparation d'un composé de formule 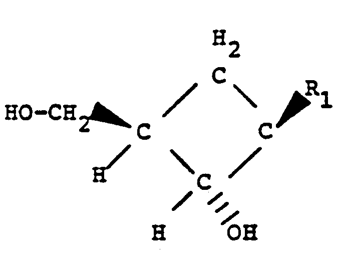 dans laquelle R₁ est 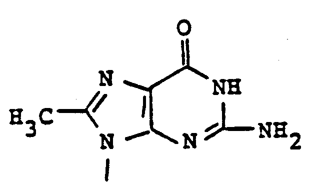 qui consiste à faire réagir un composé de formule 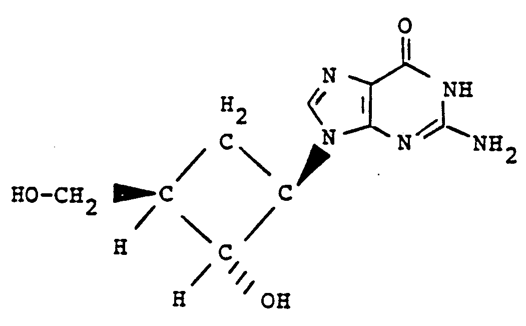 avec de l'hydroperoxyde de t-butyle dans de l'acide sulfurique aqueux, en présence de sulfate ferreux.

4. Procédé de préparation d'un composé de formule 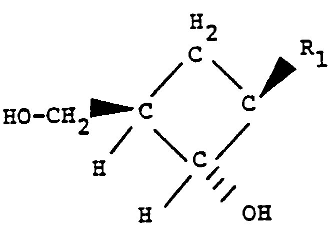 dans laquelle R₁ est 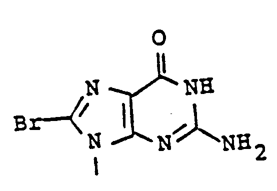 qui consiste à faire réagir un composé de formule 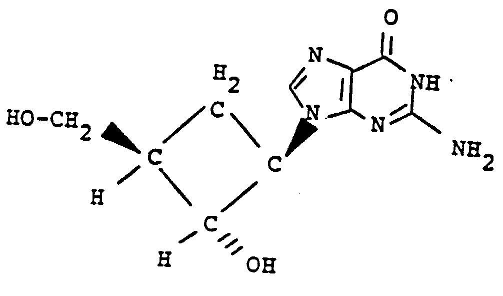 avec de l'eau de brome.

5. Procédé selon la revendication 1, dans lequel R₁ est ou

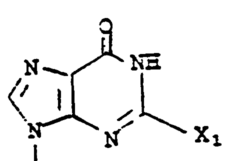

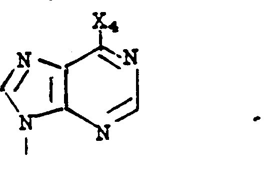

6. Procédé selon la revendication 1, dans lequel R₁ est

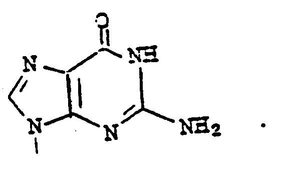

7. Procédé selon la revendication 1 ou 2, dans lequel R₁ est

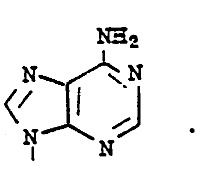

8. Procédé selon l'une quelconque des revendications 1, 6 ou 7, dans lequel R₂ et R₃ sont indépendamment l'hydrogène ou

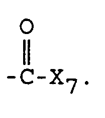

9. Procédé selon l'une quelconque des revendications 1, 6 ou 7, dans lequel R₂ et R₃ sont indépendamment l'hydrogène ou -P0₃H₂.

10. Procédé selon la revendication 9, dans lequel R₂ et R₃ sont l'hydrogène.

11. Procédé selon la revendication 1, dans lequel R₁ est

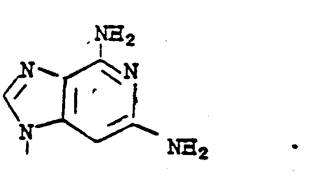

12. Procédé selon la revendication 1, dans lequel R₁ est

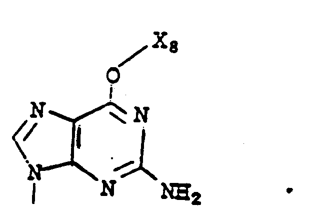

13. Procédé selon la revendication 1, dans lequel R₁ est

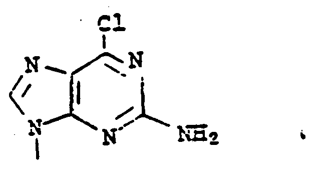

14. Procédé selon la revendication 1, dans lequel R₁ est

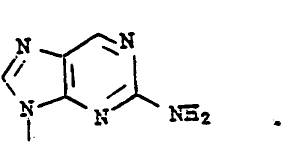

15. Procédé selon la revendication 1, dans lequel R₁ est

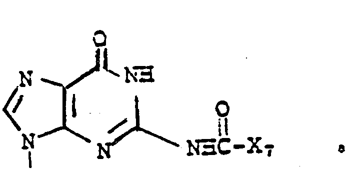

16. Procédé selon la revendication 1, dans lequel R₁ est

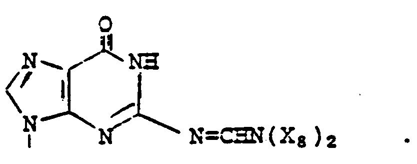

17. Procédé selon la revendication 1, dans lequel R₁ est

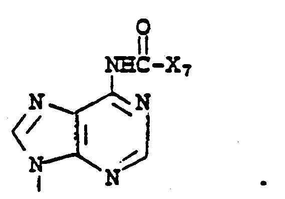

18. Procédé selon la revendication 1, dans lequel Ri est

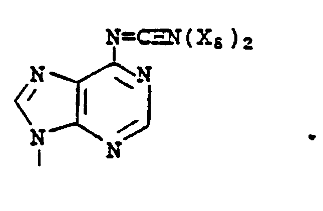

19. Procédé selon la revendication 1, dans lequel le composé préparé est la (1α,2β,3α)-9-[2-hydroxy-3-(hydroxyméthyl)cyclobutyl]guanine.

20. Procédé selon la revendication 1, dans lequel le composé préparé est le (1α,2β,3α)-3-(6-amino-9H-purin-9-yl)-2-hydroxycyclobutaneméthanol.

21. Procédé selon la revendication 1, dans lequel le composé préparé est la (1α,2β,3α)-2-amino-1,9-dihydro-9-[2-hydroxy-3-(hydroxyméthyl)cyclobutyl]-8-méthyl-6H-purin-6-one.

22. Procédé selon la revendication 1, dans lequel le composé préparé est la (1a,2ⱼ8,3a)-2-amino-8-bromo-1,9-dihydro-9-[2-hydroxy-3-(hydroxyméthyl) cyclobutyl]-6H-purin-6-one.

23. Procédé selon la revendication 1, dans lequel le composé préparé est la (1α,2β,3α)-2,8-diamino-1,9-dihydro-9-[2-hydroxy-3-(hydroxyméthyl)cyclobutyl]-6H-purin-6-one.

24. Procédé de préparation d'une composition pharmaceutique consistant à associer une quantité efficace d'un composé tel qu'il est défini dans l'une quelconque des revendications 1 à 23, à un porteur physiologiquement acceptable.

25. Procédé selon la revendication 24, dans lequel la composition pharmaceutique préparée est une composition antivirale.

26. Procédé selon la revendication 25, dans lequel la composition antivirale préparée est utilisable pour traiter une infection virale chez un hôte mammifère ou avien.

27. Procédé de préparation d'un composé ayant pour formule 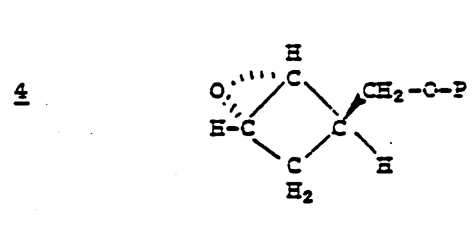 dans laquelle P est un groupe protecteur de la fonction hydroxyle, consistant en l'époxydation d'un composé de formule

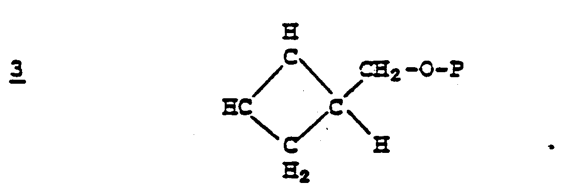

## Revendications (Revendications pour l'(les) Etat(s) contractant(s) suivant(s) : suivant : GR)

1. Composé ayant pour formule 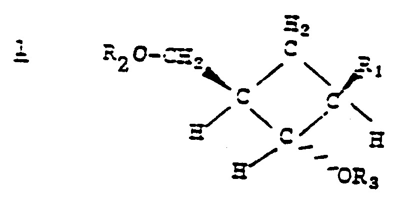 ou sel pharmaceutiquement acceptable de celui-ci, formule dans laquelle R₁ est 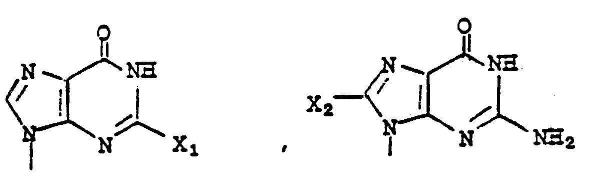 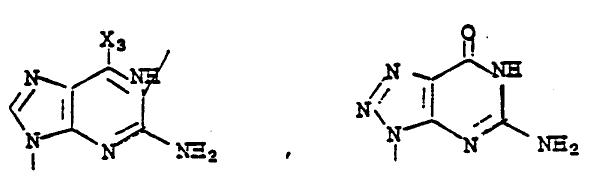 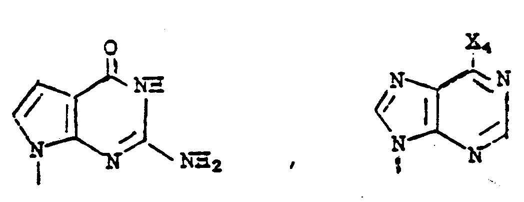 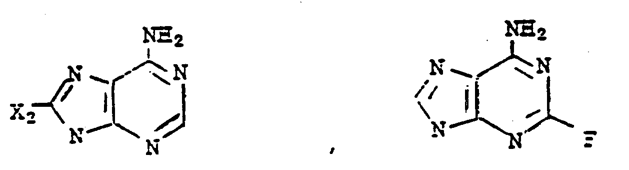 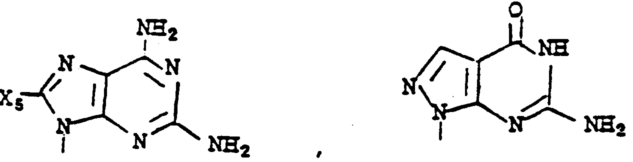 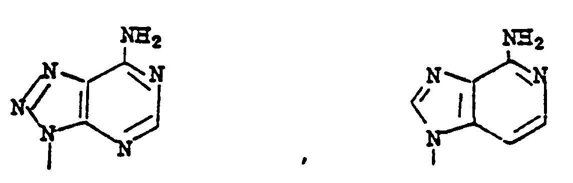 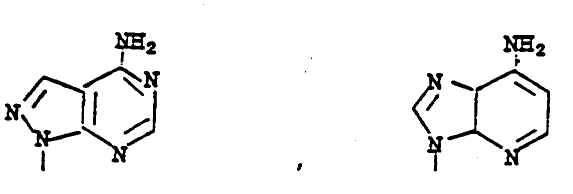 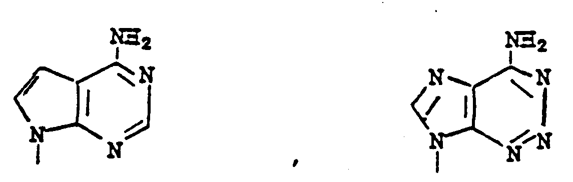 où X₁ est l'hydrogène ou un groupe amine, 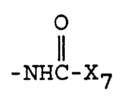 ou -N = CHN(X₈)₂,
X₂ est un groupe méthyle, un atome de fluor, de chlore, de brome ou d'iode, ou un groupe hydroxy ou amine,
X₃ est un atome d'hydrogène ou de chlore, ou O-X₈,
X₄ est un groupe amine, 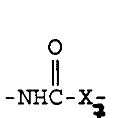 ou -N = CHN(X₈)₂,
X₅ est l'hydrogène, le groupe méthyle, un atome de fluor, de chlore, de brome ou d'iode, ou un groupe hydroxy ou amine,
X₇ est l'hydrogène ou un groupe alkyle, alkyle substitué ou aryle,
X₈ est un groupe alkyle,
R₂ et R₃ sont indépendamment l'hydrogène ou un groupe -P0₃H₂ ou 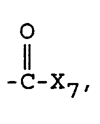 où
le terme "alkyle" désigne un groupe à chaîne droite ou ramifiée ayant de 1 à 10 atomes de carbone, le terme "alkyle substitué" désigne un groupe alkyle ayant un ou plusieurs substituants halogène, amine, azide, hydroxy, cyano, trialkylammonium (où chaque groupe alkyle a de 1 à 6 atomes de carbone), alcoxy de 1 à 6 atomes de carbone , aryle ou carboxy, le terme "aryle" désigne un groupe phényle éventuellement substitué par un, deux ou trois substituants alkyle de 1 à 6 atomes de carbone, alcoxy de 1 à 6 atomes de carbone, halogène, trifluorométhyle, amine, alkylamine, dialkylamine, nitro, cyano, alcanoyloxy de 2 à 11 atomes de carbone, carboxy, carbamoyle ou hydroxy.

2. Composé selon la revendication 1, dans lequel R₁ est 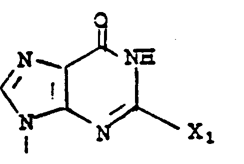 ou

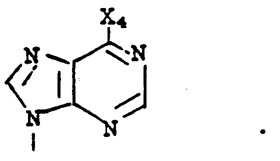

3. Composé selon la revendication 2, dans lequel R₁ est

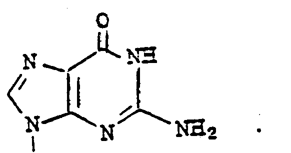

4. Composé selon la revendication 2, dans lequel R₁ est

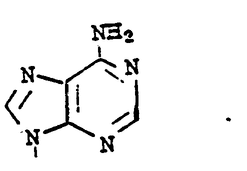

5. Composé selon l'une quelconque des revendications 1, 3 ou 4, dans lequel R₂ et R₃ sont indépendamment l'hydrogène ou

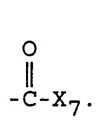

6. Composé selon l'une quelconque des revendications 1, 3 ou 4, dans lequel R₂ et R₃ sont indépendamment l'hydrogène ou -P0₃H₂.

7. Composé selon la revendication 6, dans lequel R₂ et R₃ sont l'hydrogène.

8. Composé selon la revendication 1, dans lequel R₁ est

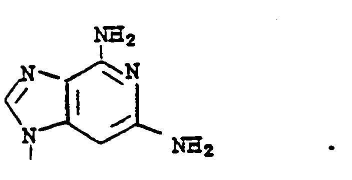

9. Composé selon la revendication 1, dans lequel R₁ est

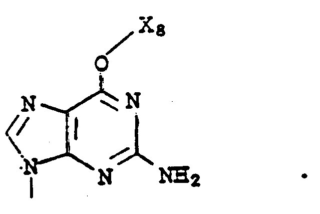

10. Composé selon la revendication 1, dans lequel R₁ est

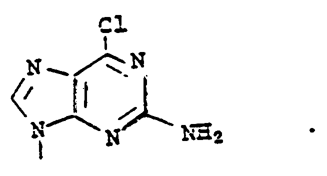

11. Composé selon la revendication 1, dans lequel R₁ est

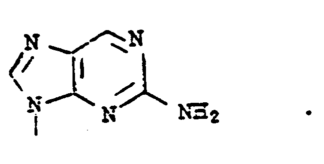

12. Composé selon la revendication 1, dans lequel Ri est

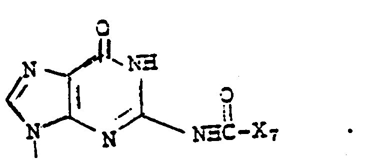

13. Composé selon la revendication 1, dans lequel R₁ est

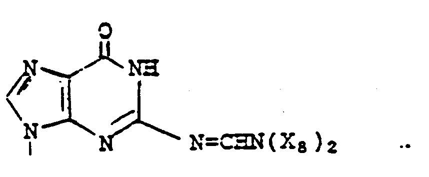

14. Composé selon la revendication 1, dans lequel R₁ est

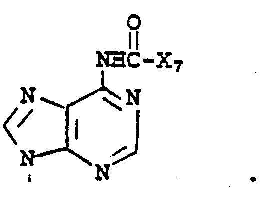

15. Composé selon la revendication 1, dans lequel R₁ est

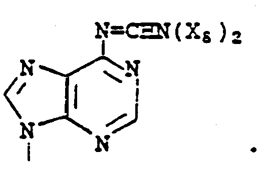

16. Composé selon la revendication 1, qui est la (1α,2β,3α)-9-[2-hydroxy-3-(hydroxyméthyl)cyclobutyl]-guanine.

17. Composé selon la revendication 1, qui est le (1α,2β,3α)-3-(6-amino-9H-purin-9-yl)-2-hydroxycyclobuta- neméthanol.

18. Composé selon la revendication 1, qui est la (1α,2β,3α)-2-amino-1,9-dihydro-9-[2-hydroxy-3-(hydroxy- méthyl)cyclobutyl]-8-méthyl-6H-purin-6-one.

19. Composé selon la revendication 1, qui est la (1α,2β,3α)-2-amino-8-bromo-1,9-dihydro-9-[2-hydroxy-3-(hydroxyméthyl)cyclobutyl]-6H-purin-6-one.

20. Composé selon la revendication 1, qui est (1α,2β,3α)-2,8-diamino-1,9-dihydro-9-[2-hydroxy-3-(hydroxy- méthyl)cyclobutyl]-6H-purin-6-one.

21. Procédé de préparation d'une composition pharmaceutique consistant à associer une quantité efficace d'un composé tel qu'il est défini dans l'une quelconque des revendications 1 à 20, à un porteur physiologiquement acceptable.

22. Procédé selon la revendication 21, dans lequel la composition pharmaceutique préparée est une composition antivirale.

23. Procédé selon la revendication 22, dans lequel la composition antivirale préparée est utilisable pour traiter une infection virale chez un hôte mammifère ou avien.

24. Composé ayant pour formule 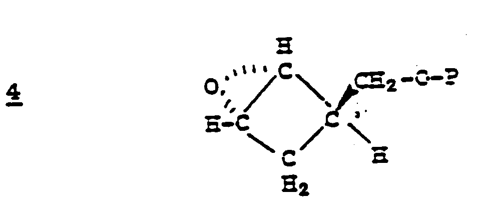 dans laquelle P est un groupe protecteur de la fonction hydroxyle.

25. Procédé de préparation de composés de formule 1 tels qu'ils sont définis dans la revendication 1, qui consiste à faire réagir un composé de formule 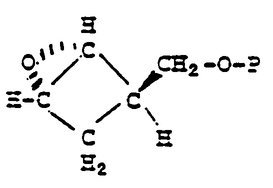 dans laquelle P est un groupe protecteur, avec un composé de formule
Rᵢ H
qui est facultativement protégé, et à éliminer le groupe protecteur pour obtenir un composé de formule 1.

26. Procédé de préparation d'un composé de formule 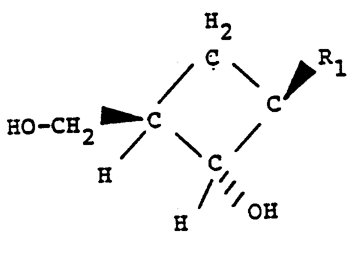 dans laquelle R₁ est 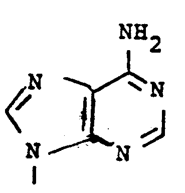 qui consiste à faire réagir un composé de formule 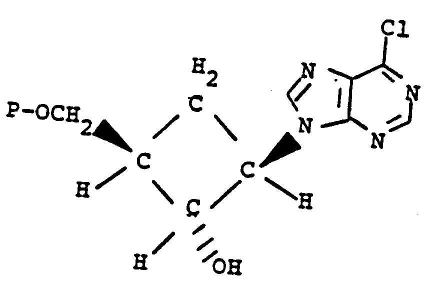 avec de l'ammoniaque méthanolique chaude, puis à éliminer le groupe protecteur P.

27. Procédé de préparation d'un composé de formule 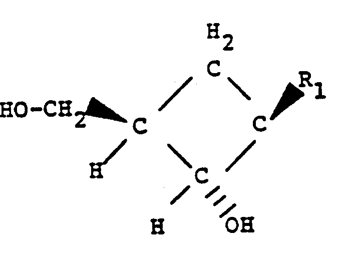 dans laquelle R₁ est 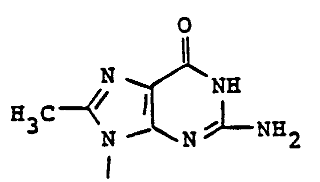 qui consiste à faire réagir un composé de formule 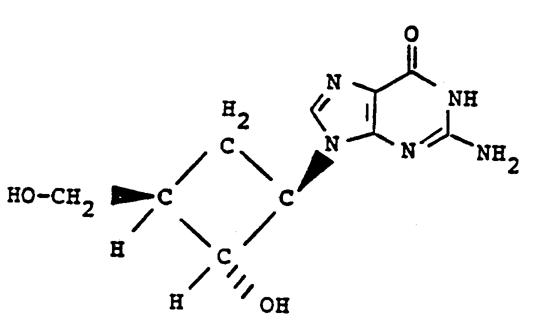 avec de l'hydroperoxyde de t-butyle dans de l'acide sulfurique aqueux, en présence de sulfate ferreux.

28. Procédé de préparation d'un composé de formule 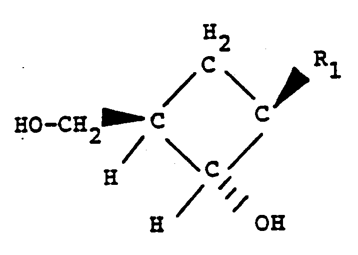 dans laquelle R₁ est 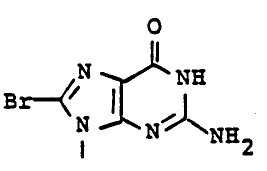 qui consiste à faire réagir un composé de formule 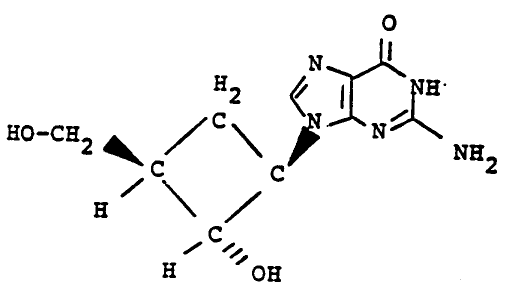 avec de l'eau de brome.
